# EUROPEAN PATENT SPECIFICATION

(11) **EP 2 214 748 B1**
(45) Date of publication and mention of the grant of the patent: **07.12.2016**
(21) Application number: 08840401.7
(22) Date of filing: 17.10.2008
(51) Int. Cl.: A61L 31/16, A61F 2/82, A61L 27/34, A61L 27/54, A61L 27/58, A61L 31/10, A61L 33/14, A61L 31/14

(54) **SELF-ELIMINATING COATINGS**
SELBSTELIMINIERENDE BESCHICHTUNGEN
REVÊTEMENTS AUTODÉGRADABLES

(30) Priority: 19.10.2007 US 999634 P
(43) Date of publication of application: 11.08.2010
(73) Proprietor: Interface Biologics Inc., Toronto, Ontario M5G 1L7 (CA)
(72) Inventor: ESFAND, Roseita, Mississauga Ontario L5K 1Y3 (CA); SANTERRE, Paul J., Whitburn Ontario L1R 1R5 (CA); ERNSTING, Mark J., Toronto, Ontario M4Y 2C7 (CA); WANG, Vivian Z., North York, Ontario M2N 7K1 (CA); TJAHYADI, Sylvia, Toronto Ontario M4Y 2C7 (CA)
(74) Representative: Lahrtz, Fritz
(86) International application number: PCT/CA2008/001848
(87) International publication number: WO 2009/049426

(56) References cited:
- WO-A2-2007/004067
- WO-A2-2007/148230
- CA-A1- 2 470 524
- CA-A1- 2 484 269
- CA-A1- 2 555 364
- US-A- 6 127 507
- US-A1- 2003 204 238
- US-B2- 6 770 725
- US-B2- 6 770 725

## Description

### Background of the Invention

The invention relates to a coating for medical devices.

Implantable medical articles can be instrumental in saving and/or enhancing the quality of the life of patients. However, a significant barrier to the use of implantable devices is the possibility of adverse reactions of the body such as thrombogenic and immune responses. Common materials used to manufacture implantable medical articles include metals, minerals or ceramics, and polymers. It is generally desirable to modify the surface of such materials in order to provide the surface with properties that are different from the properties of the material, e.g., in terms of infection resistance (i.e., via the delivery of a biologically active agent), thromboresistance, radiopacity, conductivity, and/or biocompatibility.

Various synthetic techniques have been used to impart desired chemical, physical and biological properties to materials used to manufacture implantable medical devices. One approach, for example, involves application of a parylene coating to devices, wherein a low molecular weight monomer is condensed and polymerized on a substrate, forming a matrix on the surface of the medical device. In another approach, biomaterials, e.g., heparin or albumin, are coupled directly to the surface of the medical device to reduce thrombogenicity (Nicholas A. Peppas et al., Science, 263: 1715 (1994)). Such approaches have a number of limitations. For example, a thick polymer coating, or a surface-coupled polymer coating, when applied to a medical device, such as a stent, will often have different physical properties than the underlying substrate (i.e., a metal) and, consequently, may not respond similarly to tensile, shear, or compression forces, causing the coating to crack, flake, or delaminate. Such instability can have serious adverse consequences when the coating cracks, flakes, or delaminates in vivo. This problem is exacerbated for certain medical devices, such as catheters and stents, which are subjected to deformation in vivo. When the device expands, it is important that the coating is capable of undergoing the same deformation without breaking or coming loose.

The safety of implantable devices can also be compromised by a lack of biocompatibility. Once implanted, a medical device resides in contact with tissue and may produce local inflammation, at the site of implantation, as the host responds to the implant as a foreign body. The design of a drug delivery platform that addresses the host's response to an implantable device has long been desired. Medical implants are excellent platforms for direct and localized drug delivery, however the challenge is with the polymer system used for such applications. For example, although cardiovascular stents revolutionized the management of controlling the re-narrowing of artries, stent-restenosis persists as a clinical risk factor. To address this problem, at the molecular level, a host of polymers as drug delivery platforms have been used. This was captured in the design and implantation of drug eluting stents. However the drug delivery platforms used in "drug eluting stents" are generally durable polymers and remain on the stent platform for an indefinite timeline which in turn initiates a series of un-desired host responses (Perin et al., Review in Cardiovascular Medicine, 6:S13 (2005)). Self-eliminating fluoro-polymers have been investigated as drug release coating (US6770725 and WO2007/148230). Coating of a
pharmaceutically active compound directly on a stent platform has also been investigated. Crystallinity of the deposited pharmaceutically active compound on the surface is often the result. The formation of a crystalline coating produces a dumping effect in which all of the drug on the stent is released at one time. Such a release profile is undesirable because it can result in toxicity leading to necrosis at the site of release.

New coatings that can function as drug delivery matrices, are easy to apply, biocompatible, and have a limited residency time, while allowing for the controlled release of biologically active agents, are needed to address the limitations present in the art.

### Summary of the Invention

The invention features the use of a matrix coating consisting of low molecular weight components and comprising an oligomer and a biologically active agent. The matrix coatings are self-eliminating or bioerodible upon implantation into a subject. The matrix coatings can be used to enhance biocompatibility and to control the local delivery of biologically active agents.

In a first aspect, the invention features
an implantable medical device having a surface and a matrix coating applied to said surface of said implantable medical device, said matrix coating consisting of components having a molecular weight of less than 20 kDa, said matrix coating comprising (i) an oligomer having a structure described by formula (II): F_{T} - [B - (oligo)]ₙ- B - (F_{T})_{g} (II), wherein B comprises a urethane; oligo comprises polypropylene oxide, polyethylene oxide, polycarbonate, or polytetramethyleneoxide; F_{T} is an oligofluoro group; g is 1; and n is an integer from 1 to 10; and (ii) a biologically active agent, wherein (a) said matrix coating is self-eliminating or bioerodible upon implantation into a subject, (b) said biologically active agent, when on the implantable medical device, resides solely within said matrix coating; (c) said matrix coating does not have the properties of a base polymer; (d) said matrix coating is applied to said surface by spray coating, printing, or dip coating said implantable medical device; and (e) said matrix coating thickness is from 0.05 to 15 microns.

In a related aspect, the invention features a method for making a coated implantable medical device as comprised by the present invention having a surface by coating the surface with a matrix coating
consisting of components having a molecular weight of less than 20 kDa, the matrix coating including (i) an oligomer and (ii) a biologically active agent, wherein the matrix coating is self-eliminating or bioerodible upon implantation into a subject and wherein the biologically active agent when on the implantable medical device resides solely within the matrix coating.

The biologically active agents can be selected from proteins, peptides, carbohydrates, antibiotics, antiproliferative agents, rapamycin macrolides, analgesics, anesthetics, antiangiogenic agents, antithrombotic agents, vasoactive agents, anticoagulants, immunomodulators, cytotoxic agents, antiviral agents, antibodies, neurotransmitters, psychoactive drugs, oligonucleotides, vitamins, lipids, prodrugs thereof. Biologically active agents useful in the methods and matrix coatings of the invention include any biologically active agent described herein.

In certain embodiments, the biologically active agent is simply mixed with oligomers of the matrix coating. In other embodiments, the biologically active agent is complexed to an oligomer in the matrix coating. It is disclosed that the biologically active agent can be covalently tethered to an oligomer in the matrix coating.

In still other embodiments, the biologically active agent is uniformly distributed throughout the matrix coating. For example, the biologically active agent can be dissolved in the matrix coating.

The invention discloses an implantable medical device having a surface and a matrix coating applied to the surface of the implantable medical device, the matrix coating including an oligofluorinated oligomer, wherein the matrix coating is self-eliminating or bioerodible upon implantation into a subject and wherein the oligofluorinated oligomer when on the implantable medical device resides solely within the matrix coating.

Furthermore, the invention discloses a method for making a coated implantable medical device having a surface by coating the surface with a matrix coating including an oligofluorinated oligomer, wherein the matrix coating is self-eliminating or bioerodible upon implantation into a subject and wherein the oligofluorinated oligomer when on the implantable medical device resides solely within the matrix coating.

The implantable medical devices of the invention can have a surface material selected from, for example, metals, metal alloys, ceramics, base polymers, and glasses. Implantable medical devices that can be coated using the methods and matrix coatings of the invention include, without limitation, cardiac-assist devices, catheters, stents, prosthetic implants, artificial sphincters, drug delivery devices, and any other implantable devices described herein. In certain embodiments, the implantable medical device is a stent.

The invention further features a stent having a surface and a matrix coating applied to the surface of the stent, the matrix coating consisting of components having a molecular weight of less than 20 kDa, the matrix coating including (i) an oligomer and (ii) a biologically active agent selected from antiproliferative agents and rapamycin macrolides, wherein the matrix coating is self-eliminating or bioerodible upon implantation into a subject and wherein the biologically active agent when on the stent resides solely within the matrix coating. The stent has the features as referred to above under the first aspect of the present invention.

The invention also discloses a stent having a surface and a matrix coating applied to the surface of the stent, the matrix coating including an oligofluorinated oligomer, wherein the matrix coating is self-eliminating or bioerodible upon implantation into a subject and wherein the oligofluorinated oligomer when on the stent resides solely within the matrix coating. In certain embodiments, the matrix coating further includes a biologically active agent selected from antiproliferative agents and rapamycin macrolides.

Antiproliferative agents which can be used on the coated stents of the invention include, without limitation, methotrexate, trimetrexate, gemcitabine, vinblastine, vincristine, etoposide, teniposide, topotecan, irinotecan, camptothecin, 9-aminocamptothecin, paclitaxel, docetaxel, daunorubicin, doxorubicin, dactinomycin, idarubicin, bleomycin, tamoxifen, and any other antiproliferative agent described herein.

Rapamycin macrolides which can be used on the coated stents of the invention include, without limitation, rapamycin, CCI-779, Everolimus, ABT-578, and any other rapamycin macrolide described herein.

In a related aspect, the invention features the stent as comprised by the present invention for inhibiting restenosis at a site in a vessel. This may occur by implanting a stent of the invention at the site.

The invention further features the implantable medical device as comprised by the present invention for delivering a biologically active agent to a subject. This may occur by implanting into the subject an implantable medical device having a matrix coating of the invention, where the coating matrix includes a biologically active agent.

Oligomers that can be used in the methods and matrix coatings of the invention include polyurethanes, polyureas, polyamides, polyalkylene oxides, polycarbonates, polyesters, polylactones, polysilicones, polyethersulfones, polyolefins, polyvinyls, polypeptides, polysaccharides, and combinations thereof. However, only polypropylene oxide, polyethylene oxide, polycarbonate or polytetramethylene oxide are comprised by the methods and matrix coatings of the invention.

As comprised by the present invention, the oligomer in the matrix coating is an oligofluorinated oligomer. The oligofluorinated oligomers can be any described herein. The oligofluorinated oligomer is described by formula (I) (not comprised by the present invention):

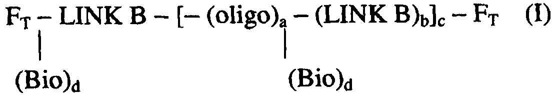

In formula (I), oligo is an oligomeric segment; Bio is a biologically active agent; F_{T} is an oligofluoro group; each Link B is, independently, an organic moiety covalently bound to oligo, F_{T}, or Bio; a is an integer greater than 0; b and c are each, independently, integers greater than or equal to 0; and d is 0 or 1. As comprised by the present invention, the oligofluorinated oligomer is described by formula (II):

**F_{T} - [B - (oligo)]ₙ- B - (F_{T})_{g}** **(II)**

In formula (II), B includes a urethane; oligo includes polycarbonate, polypropylene oxide, polyethylene oxide, or polytetramethyleneoxide; F_{T} is an oligofluoro group; g is 1; and n is an integer from 1 to 10. g may be 0 which is, however, not comprised by the present invention.

Oligofluoro groups include, without limitation, groups having the formula:

CF₃(CF₂)ₚX

,

(CF₃)₂CF(CF₂)ₚX,

or

(CF₃)₃C(CF₂)ₚX,

wherein X is CH₂CH₂-, (CH₂CH₂O)ₙ, CH₂CH(OH)CH₂O-,
CH₂CH(CH₂OH)O-, or a bond; p is an integer between 2 and 20; and n is an integer between 1 and 10.

In any of the above methods and coatings of the invention, the matrix coating can consist of components having a molecular weight of less than

**18 kDa, 16 kDa, 14 kDa, 12 kDa, 10 kDa, 9 kDa, 8 kDa, 7 kDa, 6 kDa, 5 kDa, 4 kDa, or even 3 kDa.** Components having a molecular weight of less than 40 kDa, 35 kDa, 30 kDa, or 25 kDa can occur within the matrix coating, however, are not comprised by the present invention.

The coatings of the invention can by applied by brushing, printing, spraying, wiping, or dipping the surface with the matrix coating. In certain embodiments, the step of coating includes dissolving the constituents of the matrix coating in a solvent to form a solution and applying the solution to the surface of the implantable medical device. In still other embodiments, the step of coating includes mixing the constituents of the matrix with a diluent to form a fluid mixture and applying the fluid mixture to the surface of the implantable medical device.

In any of the above methods and coatings of the invention, the matrix coating has a thickness of from 0.05 to 15 microns, although the matrix coating can also have a thickness of from 0.1 to 15 microns, 0.1 to 10 microns, 0.1 to 5 microns, 0.1 to 3 microns, or even 0.1 to 1 microns, which thicknesses are, however, not comprised by the present invention.

In any of the above methods and coatings of the invention, the uncoated implantable medical device is coated to produce a coated implantable medical device, the coated implantable medical device having, upon implantation into an animal, reduced protein deposition, reduced fibrinogen deposition, reduced platelet deposition, or reduced inflammatory cell adhesion in comparison to said uncoated implantable medical device.

**By "base polymer" is meant a self supporting polymer having a tensile strength of** from about 350 to about 10,000 psi, elongation at break from about 300% to about 1500%, an unsupported thickness of from about 5 to about 100 microns, and a supported thickness of from about 1 to about 100 microns.

As used herein, "LinkB" refers to a coupling segment capable of covalently linking oligomers, biologically active agents, and/or oligofluoro groups. Typically, LinkB molecules have molecular weights ranging from 40 to 700. Preferably the LinkB molecules are selected from the group of functionalized diamines, diisocyanates, disulfonic acids, dicarboxylic acids, diacid chlorides and dialdehydes, wherein the functionalized component has secondary functional chemistry that is accessed for chemical attachment of an oligofluoro group. Such secondary groups include, for example, esters, carboxylic acid salts, sulfonic acid salts, phosphonic acid salts, thiols, vinyls and secondary amines. Terminal hydroxyls, amines or carboxylic acids on the oligo intermediates can react with diamines to form oligo-amides; react with diisocyanates to form oligo-urethanes, oligo-ureas, oligo-amides; react with disulfonic acids to form oligo-sulfonates, oligo-sulfonamides; react with dicarboxylic acids to form oligo-esters, oligo-amides; react with diacid chlorides to form oligo-esters, oligo-amides; and react with dialdehydes to form oligo-acetal, oligo-imines.

By "oligo" or "oligomer" is meant a relatively short length of a repeating unit or units, generally less than about 50 monomeric units and molecular weights less than 10,000 but preferably <5,000 Daltons. Preferably, oligo is selected from the group consisting of polyurethane, polyurea, polyamides, polyalkylene oxide, polycarbonate, polyester, polylactone, polysilicone, polyethersulfone, polyolefin, polyvinyl, polypeptide, polysaccharide; and ether and amine linked segments thereof.

By "prodrug" is meant a precursor to a biologically active agent which is converted in vivo, e.g., by enzymatic and/or hydrolytic mechanisms, into a biologically active agent. Prodrugs include, without limitation, esterified biologically active agents. Prodrugs useful in the methods and compositions of the invention include, for example, biologically active agents covalently tethered via a hydrolyzable linkage to an oligomer in a matrix coating of the invention.

As used herein, "self-eliminating" refers to the diffusion of a matrix coating from the surface of an implantable medical device. Self-eliminating coatings are those in which greater than 70%, 80%, or even 90% (w/w) of the coating diffuses from the surface over a period of less than 2 months, 1 month, or 15 days under flow conditions in buffer, artificial urine, or plasma as provided in the Examples. It is understood that the self-elimination kinetics of any matrix coating on any particular implantable device will vary with the shape of the device, the constituents of the matrix, and the site of implantation. Of importance is that the matrix coatings of the invention are designed to be transitory in nature, leaving the original uncoated implantable medical device intact at the site of implantation.

As used herein, "bioerodible" refers to the diffusion of a matrix coating from the surface of an implantable medical device. Bioerodible coatings are those in which greater than 30%, 40%, or 50% (w/w) of the coating diffuses from the surface over a period of less than 6 months, 4 months, 3 months, 2 months, or 1 month under sink conditions in buffer, artificial urine, or plasma as provided in the Examples. It is understood that the self-elimination kinetics of any matrix coating on any particular implantable device will vary with the shape of the device, the constituents of the matrix, and the site of implantation. Of importance is that the matrix coatings of the invention are designed to be transitory in nature, leaving the original uncoated implantable medical device intact at the site of implantation.

As used herein, "covalently tethered" refers to moieties separated by one or more covalent bonds. For example, where an oligofluoro group is covalently tethered to an oligomer, tethered includes the moieties separated by a single bond as well as both moieties separated by, for example, a LinkB segment to which both moieties are covalently attached.

As used herein, the term "oligofluorinated" refers to an oligomer covalently linked to an oligofluoro group for use in a matrix coating of the invention.

As used herein, "complexed" or "complexation" refers to an interaction, either non-covalent or via coordination to a metal center, between a complexing moiety in an oligomer contained within a matrix coating of the invention and a biologically active agent. Examples of non-covalent bonding interactions which can be used in accordance with the present invention include, without limitation, hydrogen bonding, ionic interactions (e.g., dipole-dipole interactions, ion pairing, and salt formation), inclusion complexes, clathration, van der Waals interactions (e.g., pi-pi stacking), and combinations thereof. The interaction can also be via coordination to a metal center by both the complexing moiety and the biologically active agent. In some instances, the biologically active agent includes a metal center which is coordinated to the complexing moiety.

As used herein, "complexing moiety' refers to certain embodiments of the invention including a portion of an oligomer contained within a matrix coating of the invention which complexes a biologically active agent either via a non-covalent interaction or coordination to a metal center, forming a polymer complex. The complexing moiety can be a charged moiety, e.g., a moiety which loses a proton at physiological pH thereby becoming negatively charged (e.g., carboxylate, or phosphodiester), a moiety which gains a proton at physiological pH thereby becoming positively charged (e.g., ammonium, guanidinium, or amidinium), a moiety that includes a net formal positive charge without protonation (e.g., quaternary ammonium), or a moiety that includes a net formal negative charge without loss of a proton (e.g., borate, BR₄⁻). Exemplary charged complexing moieties include, without limitation, carboxylate, phosphodiester, phosphoramidate, borate, phosphate, phosphonate, phosphonate ester, sulfonate, sulfate, thiolate, phenolate, ammonium, amidinium, guanidinium, quaternary ammonium, and imidazolium functionalities. The complexing moiety can be designed to physically encapsulate, in whole or in part, the biologically active agent, such as a cyclodextrin. The complexing moiety be designed to ligate a complementary oligonucleotide and/or peptide sequence present in the biologically active agent. The complexing moiety can be designed to coordinate a metal center including the biologically active agent, either as a ligand alone or including the metal center.

A description of how make complexing moieties and complexation with biologically active agents is described in U.S. Patent Publication No. 20070037891.

Other features and advantages of the invention will be apparent from the following Detailed Description, the Drawings, and the Claims.

### Brief Description of the Drawings

Figure 1 is a 3D HPLC chromatogram of Compound 3, showing a pure material with no side products.
Figure 2 is an SEM image of a stent coated with Compound 1, showing thin coverage with minimal webbing.
Figure 3 is an SEM image of a stent coated with Compound 1, crimped and deployed in buffer, showing thin coverage.
Figure 4 is an SEM image of a stent coated with Compound 2, showing thin coverage with minimal webbing.
Figure 5 is a confocal / fluorescent microscopy (5X Fluor lens and a DAPI UV filter) image of a stent coated with Compound 3, indicating the presence of Compound 3 on the stent surface.
Figure 6 is a confocal / fluorescent microscopy (5X Fluor lens and a DAPI UV filter) image of a crimped stent coated with Compound 3, indicating the presence of Compound 3 on the stent surface.
Figure 7 is a confocal / fluorescent microscopy (5X Fluor lens and a DAPI UV filter) image of a deployed stent coated with Compound 3, indicating the presence of Compound 3 on the stent surface.
Figure 8 is an SEM image of a stent coated with Compound 4, indicating a thin coating.
Figure 9 is an SEM image of a stent coated with Compound 5, indicating a thin coating.
Figure 10 is an SEM image of a stent coated with Compound 6, indicating a thin coating.
Figure 11 is an SEM image of a stent coated with Compound 7, indicating a thin coating.
Figure 12 is an SEM image of a stent coated with Compound 8, indicating a thin coating.
Figure 13 is an SEM image of a stent coated with Compound 9, indicating a thin coating.
Figure 14 is an SEM image of a stent coated with Compound 10, indicating a thin coating.
Figure 15 is an SEM image of a stent coated with Compound 11, indicating a thin coating.
Figure 16 is an SEM image of a stent coated with Compound 12, indicating a thin coating.
Figure 17 is an SEM image of a stent coated with Compound 14, indicating a thin coating.
Figure 18 is an SEM image of a stent coated with Compound 15, indicating a thin coating.
Figure 19 is an SEM image of a stent coated with Compound 1 and Compound 7, indicating a thin coating.
Figure 20 is an SEM image of a stent coated with Compound 2 + 8.8 wt% PTX, indicating a thin coating.
Figure 21 is an SEM image of a stent coated with Compound 6 + 8.8 wt% PTX, indicating a thin coating.
Figure 22 is a plot of PTX release from Compound 1, 2, and 6 + 5, 8.8 and 20 wt% PTX into Tween PBS at 37°C (films cast in vials), showing the effect of Compound and PTX concentration on release.
Figure 23 is a plot of PTX release from Compound 1 and 6 + 5 and 8.8 wt% PTX into Tween PBS at 20°C (films cast in vials), showing effect of Compound and PTX concentration on release.
Figure 24 is a plot of PTX release from Compound 1 and 6 + 5 and 8.8 wt% PTX into Tween PBS at 37°C (films cast in vials), showing effect of Compound and PTX concentration on release.
Figure 25 is an SEM image of a stent coated with Compound 1 + 8.8 wt% PTX, showing good coverage.
Figure 26 is a series of SEM images of Compounds + 1 wt% PTX coated on stents, showing good coverage: (a) Compound 1, (b) Compound 6, (c) Compound 7, (d) Compound 1+7, (e) Compound 8, (f) Compound 9, (g) Compound 12.
Figure 27 is a picture of a Compound 1 + 8.8 wt% PTX coated coupon secured to cardiac muscle, showing contact between the two substrates.
Figure 28 is a picture of a coupon coated with Compound 1 + 8.8 wt% PTX after contact with cardiac muscle, showing the appearance of the coating.
Figure 29 is a plot of PTX remaining on the stainless steel coupons coated with Compound 1 + 8.8% PTX in contact with cardiac muscle, showing the PTX release profile.
Figure 30 is a plot of PTX remaining on the stainless steel coupons coated with Compound 1 + 1% PTX in contact with cardiac muscle, showing the PTX release profile.
Figure 31 is a plot of the residency of Compounds 1, 2, 3, 6, 7, 1+7, 8, 9, 10, 11, 12, 14, 15, and 16 in a PBS sink condition, showing the effect of formulation on residency time.
Figure 32 is a plot of the residency of Compounds 1, 2, 6, 7, 1+7, 10, and 12 on stainless steel coupons under sink condition in PBS, showing the effect of formulation on residency time.
Figure 33 is a plot of the residency of Compounds 1, 2, 6, 7, 1+7, 10, and 12 (vials) in a porcine blood sink condition, showing the effect of formulation on residency time.
Figure 34 is a plot of the residency of Compounds 1, 2, 10, and 12 on stainless steel coupons under sink condition in porcine blood, showing the effect of formulation on residency time.
Figure 35 is a plot of the residency of Compounds 1, 2, 6, 7, 1+7, 10 and 12 in artificial urine sink condition, showing the effect of formulation on residency time.
Figure 36 is a plot of the residency of Compounds 1, 2, 4, and 6 in PBS flow condition, showing the effect of formulation on residency time.
Figure 37 is a plot of the residency of Compounds 1, 2 and 6 in porcine blood, flow condition, showing the effect of formulation on residency time.
Figure 38 is a plot of the residency of Compounds 1, 2, and 6 in artificial urine, flow condition, showing the effect of formulation on residency time.
Figure 39 is a picture of the porcine hearts used for the residency time study of Compounds 1, 2, 4 and 6. The image shows incision size and position.
Figure 40 is a plot of a long term residency study of Compounds 1, 2, 3, 6, 7, 1+7, 8,9, 10, 11, 12, 14, 15, and 16 in PBS sink condition, showing the ability to fine-tune the delivery time.
Figure 41 is a plot of mass loss for Compounds 1, 5, 7, and DL-PLGA after incubation in various solutions, showing the effect of media.
Figure 42 contains SEM images of stainless steel coupons coated with (a) Compound 1, (b) Compound 6, (c) Compound 7, (d) Compound 1+7, (e) Compound 8, (f) Compound 9, (g) Compound 10, (h) Compound 11, (i) Compound 12, (j) Compound 14 and (k) Compound 15, showing smooth coverage.
Figure 43 contains pictures of coated stainless steel coupons before and after contact with porcine blood, showing no appreciable changes to the coatings: (a) Compound 1, (b) Compound 6, (c) Compound 7, (d) Compound 1+7, (e) Compound 8, (f) Compound 9, (g) Compound 10, (h) Compound 11, (i) Compound 12, (j) Compound 14 and (k) Compound 15.
Figure 44 is a plot of U937 cell interaction with Compounds 1, 2, 6, 7, 1+7, 8, 9, 10, 11, 12, 15 and 16, showing the ability to minimize macrophage adhesion.
Figure 45 is a plot of HCAEC migration through membranes coated with Compound 1, Compound 1 + PTX, and SIBS, demonstrating good migration of HCAEC through membranes coated with Compound 1.
Figure 46 is a plot of platelet and fibrinogen interactions with sprayed films of Compounds 1, 7, 1+7 and 12, showing reduction in platelet adhesion and fibrinogen adsorption.
Figure 47 contains histology images of vessel cross-sections from two animals (A and B), showing biocompatibility profile.
Figure 48 contains SEM images of a stent coated with Compound 3 at day 0 (left) and day 42 (right), showing the stability of the coatings in storage.
Figure 49 contains SEM images of stents coated with Compound 3 after 24 hours (left) and 7 days (right) exposure to porcine blood, showing the coating profile.
Figure 50 is an image of a Compound 3 coated stent explanted from a porcine LCX artery, showing contact between the vessel wall and the stent.

### Detailed Description

The methods and compositions of the invention feature a matrix coating consisting of low molecular weight components and comprising an oligomer having a structure described by formula (II) and a biologically active agent.

The matrix coatings are self- eliminating or bioerodible upon implantation into a subject. The matrix coatings can be used to enhance biocompatibility and to control the local delivery of biologically active agents.

### Oligomers

The matrix coating of the invention includes an oligomer having a structure described by formula (II). The invention discloses as oligomers polyurethanes, polyureas, polyamides, polyaklylene oxides, polycarbonates, polyesters, polylactones, polysilicones, polyethersulfones, polyolefins, polyvinyl derivatives, polypeptides, polysaccharides, polysiloxanes, polydimethylsiloxanes, polyethylene-butylene, polyisobutylenes, polybutadienes, polypropylene oxides, polyethylene oxides, polytetramethyleneoxides, polyethylenebutylenes, polycaprolactone, polylactic, polyethylene glycol, polypropylene glycol, polydiethyleneglycol phthalate, polydiethyleneglycol adipate, polyhydroxybutyrate, polyhydroxyoctanoate, **polyhydroxyvalerate, biOH^{™} soybean oil-derivative (Cargill), and combinations and mixtures thereof.** However, formula (II) comprises polypropylene oxide, polyethylene oxide, polycarbonate or polytetramethylene oxide.

The matrix coating may optionally contain an oligomer complexed, or covalently tethered (not specifically comprised by the present invention), to a biologically active agent, or applied in a mixture including a biologically active agent.

The amount of biologically active agent loaded into the coating will depend upon the design of the oligomer in combination with the desired release profile. The oligomer may be designed for the particular agent being delivered and to provide the
biocompatibility necessary for a particular application.

The process by which the polymer complex is formed may be a two or multi-step procedure. In general, oligofluorinated oligomers used in the methods and compositions of the invention can be prepared as described in U.S. Patent No. 6,127,507, and U.S.S.N. 11/404,290.

### Biologically Active Agents

Biologically active agents can be incorporated in the coatings of the invention. The incorporation can be achieved either by mixing the matrix coating components and the biologically active agent together and applying the mixture to the surface of the article prior to implantation. In some instances, the biologically active agent is
complexed to an oligomer in the matrix coating. A detailed description of how biologically active agents may be covalently tethered or complexed to an oligofluorinated oligomer is provided in U.S. Patent No. 6,770,725 and U.S.S.N. 11/404,290. Biologically active agents that can be used in the methods and compositions of the invention include therapeutic, diagnostic, and prophylactic agents. They can be naturally occurring compounds, synthetic organic compounds, or inorganic compounds. Biologically active agents that can be used in the methods and compositions of the invention include, but are not limited to, proteins, peptides, carbohydrates, antibiotics, antiproliferative agents, rapamycin macrolides, analgesics, anesthetics, antiangiogenic agents, vasoactive agents, anticoagulants, immunomodulators, cytotoxic agents, antiviral agents, antithrombotic drugs, such as terbrogrel and ramatroban, antibodies, neurotransmitters, psychoactive drugs, oligonucleotides, vitamins, lipids, and any biologically active agent described herein.

Exemplary therapeutic agents include growth hormone, for example human growth hormone, calcitonin, granulocyte macrophage colony stimulating factor (GMCSF), ciliary neurotrophic factor, and parathyroid hormone. Other specific therapeutic agents include parathyroid hormone-related peptide, somatostatin, testosterone, progesterone, estradiol, nicotine, fentanyl, norethisterone, clonidine, scopolomine, salicylate, salmeterol, formeterol, albeterol, valium, heparin, dermatan, ferrochrome A, erythropoetins, diethylstilbestrol, lupron, estrogen estradiol, androgen halotestin, 6-thioguanine, 6-mercaptopurine, zolodex, taxol, lisinopril/zestril, streptokinase, aminobutytric acid, hemostatic aminocaproic acid, parlodel, tacrine, potaba, adipex, memboral, phenobarbital, insulin, gamma globulin, azathioprine, papein, acetaminophen, ibuprofen, acetylsalicylic acid, epinephrine, flucloronide, oxycodone percoset, dalgan, phreniline butabital, procaine, novocain, morphine, oxycodone, aloxiprin, brofenac, ketoprofen, ketorolac, hemin, vitamin B-12, folic acid, magnesium salts, vitamine D, vitamin C, vitamin E, vitamin A, Vitamin U, vitamin L, vitamin K, pantothenic acid, aminophenylbutyric acid, penicillin, acyclovir, oflaxacin, amoxicillin, tobramycin, retrovior, epivir, nevirapine, gentamycin, duracef, ablecet, butoxycaine, benoxinate, tropenzile, diponium salts, butaverine, apoatropine, feclemine, leiopyrrole, octamylamine, oxybutynin, albuterol, metaproterenol, beclomethasone dipropionate, triamcinolone acetamide, budesonide acetonide, ipratropium bromide, flunisolide, cromolyn sodium, ergotamine tartrate, and protein or peptide drugs such as TNF antagonists or interleukin antagonists. For example, the biologically active agent can be an antiinflammatory agent, such as an NSAID, corticosteriod, or COX-2 inhibitor, e.g., rofecoxib, celecoxib, valdecoxib, or lumiracoxib.

Exemplary diagnostic agents include imaging agents, such as those that are used in positron emission tomography (PET), computer assisted tomography (CAT), single photon emission computerized tomography, X-ray, fluoroscopy, and magnetic resonance imaging (MRI). Suitable materials for use as contrast agents in MRI include gadolinium chelates, as well as iron, magnesium, manganese, copper, and chromium chelates. Examples of materials useful for CAT and X-rays include iodine based materials.

A preferred biologically active agent is a substantially purified peptide or protein. Proteins are generally defined as consisting of 100 amino acid residues or more; peptides are less than 100 amino acid residues. Unless otherwise stated, the term protein, as used herein, refers to both proteins and peptides. The proteins may be produced, for example, by isolation from natural sources, recombinantly, or through peptide synthesis. Examples include growth hormones, such as human growth hormone and bovine growth hormone; enzymes, such as DNase, proteases, urate oxidase, alronidase, alpha galactosidase, and alpha glucosidase; antibodies, such as trastuzumab.

### Rapamycin Macrolides

Rapamycin (Sirolimus) is an immunosuppressive lactam macrolide that is produced by *Streptomyces hygroscopicus.* See, for example, McAlpine, J. B., et al., J. Antibiotics 44: 688 (1991); Schreiber, S. L., et al., J. Am. Chem. Soc. 113: 7433 (1991); and U.S. Patent No. 3,929,992. Exemplary rapamycin macrolides which can be used in the methods and compositions of the invention include, without limitation, rapamycin, CCI-779, Everolimus (also known as RAD001), and ABT-578. CCI-779 is an ester of rapamycin (42-ester with 3-hydroxy-2-hydroxymethyl-2-methylpropionic acid), disclosed in U.S. Patent No. 5,362,718. Everolimus is an alkylated rapamycin (40-O-(2-hydroxyethyl)-rapamycin, disclosed in U.S. Patent No. 5,665,772.

### Antiproliferative Agents

Exemplary antiproliferative agents which can be used in the methods and compositions of the invention include, without limitation, mechlorethamine, cyclophosphamide, iosfamide, melphalan, chlorambucil, uracil mustard, estramustine, mitomycin C, AZQ, thiotepa, busulfan, hepsulfam, carmustine, lomustine, semustine, streptozocin, dacarbazine, cisplatin, carboplatin, procarbazine, methotrexate, trimetrexate, fluouracil, floxuridine, cytarabine, fludarabine, capecitabine, azacitidine, thioguanine, mercaptopurine, allopurine, cladribine, gemcitabine, pentostatin, vinblastine, vincristine, etoposide, teniposide, topotecan, irinotecan, camptothecin, 9-aminocamptothecin, paclitaxel, docetaxel, daunorubicin, doxorubicin, dactinomycin, idarubincin, plicamycin, mitomycin, amsacrine, bleomycin, aminoglutethimide, anastrozole, finasteride, ketoconazole, tamoxifen, flutamide, leuprolide, goserelin, Gleevec^{™} (Novartis), leflunomide (Pharmacia), SU5416 (Pharmacia), SU6668 (Pharmacia), PTK787 (Novartis), Iressa^{™} (AstraZeneca), Tarceva^{™}, (Oncogene Science), trastuzumab (Genentech), Erbitux^{™} (ImClone), PKI166 (Novartis), GW2016 (GlaxoSmithKline), EKB-509 (Wyeth), EKB-569 (Wyeth), MDX-H210 (Medarex),2C4 (Genentech), MDX-17 447 (Medarex), ABX-EGF (Abgenix), CI-1033 (Pfizer), Avastin^{™} (Genentech), IMC-1C11 (ImClone), ZD4190 (AstraZeneca), ZD6474 (AstraZeneca), CEP-701 (Cephalon), CEP-751 (Cephalon), MLN518 (Millenium), PKC412 (Novartis), 13-cis-retinoic acid, isotretinoin, retinyl palmitate, 4-(hydroxycarbophenyl) retinamide, misonidazole, nitracrine, mitoxantrone, hydroxyurea, L-asparaginase, interferon alfa, AP23573, Cerivastatin, Troglitazone, CRx-026DHA-paclitaxel, Taxoprexin, TPI-287, Sphingosine-based lipids, and mitotane.

### Corticosteroids

Exemplary corticosteroids which can be used in the methods and compositions of the invention include, without limitation, 21-acetoxypregnenolone, alclomerasone, algestone, amcinonide, beclomethasone, betamethasone, betamethasone valerate, budesonide, chloroprednisone, clobetasol, clobetasol propionate, clobetasone, clobetasone butyrate, clocortolone, cloprednol, corticosterone, cortisone, cortivazol, deflazacon, desonide, desoximerasone, dexamethasone, diflorasone, diflucortolone, difluprednate, enoxolone, fluazacort, flucloronide, flumethasone, flumethasone pivalate, flunisolide, flucinolone acetonide, fluocinonide, fluorocinolone acetonide, fluocortin butyl, fluocortolone, fluorocortolone hexanoate, diflucortolone valerate, fluorometholone, fluperolone acetate, fluprednidene acetate, fluprednisolone, flurandenolide, formocortal, halcinonide, halometasone, halopredone acetate, hydrocortamate, hydrocortisone, hydrocortisone acetate, hydrocortisone butyrate, hydrocortisone phosphate, hydrocortisone 21-sodium succinate, hydrocortisone tebutate, mazipredone, medrysone, meprednisone, methylprednicolone, mometasone furoate, paramethasone, prednicarbate, prednisolone, prednisolone 21-diedryaminoacetate, prednisolone sodium phosphate, prednisolone sodium succinate, prednisolone sodium 21-m-sulfobenzoate, prednisolone sodium 21-stearoglycolate, prednisolone tebutate, prednisolone 21-trimethylacetate, prednisone, prednival, prednylidene, prednylidene 21-diethylaminoacetate, tixocortol, triamcinolone, triamcinolone acetonide, triamcinolone benetonide and triamcinolone hexacetonide. Structurally related corticosteroids having similar anti-inflammatory properties are also intended to be encompassed by this group.

### NSAIDs

Exemplary non-steroidal antiinflammatory drugs (NSAIDs) which can be used in the methods and compositions of the invention include, without limitation, naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid (salsalate), fenoprofen, flurbiprofen, ketoprofen, meclofenamate sodium, meloxicam, oxaprozin, sulindac, and tolmetin.

### Analgesics

Exemplary analgesics which can be used in the methods and compositions of the invention include, without limitation, morphine, codeine, heroin, ethylmorphine, O-carboxymethylmorphine, O-acetylmorphine, hydrocodone, hydromorphone, oxymorphone, oxycodone, dihydrocodeine, thebaine, metopon, ethorphine, acetorphine, diprenorphine, buprenorphine, phenomorphan, levorphanol, ethoheptazine, ketobemidone, dihydroetorphine and dihydroacetorphine.

### Antimicrobials

Exemplary antimicrobials which can be used in the methods and compositions of the invention include, without limitation, penicillin G, penicillin V, methicillin, oxacillin, cloxacillin, dicloxacillin, nafcillin, ampicillin, amoxicillin, carbenicillin, ticarcillin, mezlocillin, piperacillin, azlocillin, temocillin, cepalothin, cephapirin, cephradine, cephaloridine, cefazolin, cefamandole, cefuroxime, cephalexin, cefprozil, cefaclor, loracarbef, cefoxitin, cefmatozole, cefotaxime, ceftizoxime, ceftriaxone, cefoperazone, ceftazidime, cefixime, cefpodoxime, ceftibuten, cefdinir, cefpirome, cefepime, BAL5788, BAL9141, imipenem, ertapenem, meropenem, astreonam, clavulanate, sulbactam, tazobactam, streptomycin, neomycin, kanamycin, paromycin, gentamicin, tobramycin, amikacin, netilmicin, spectinomycin, sisomicin, dibekalin, isepamicin, tetracycline, chlortetracycline, demeclocycline, minocycline, oxytetracycline, methacycline, doxycycline, erythromycin, azithromycin, clarithromycin, telithromycin, ABT-773, lincomycin, clindamycin, vancomycin, oritavancin, dalbavancin, teicoplanin, quinupristin and dalfopristin, sulphanilamide, para-aminobenzoic acid, sulfadiazine, sulfisoxazole, sulfamethoxazole, sulfathalidine, linezolid, nalidixic acid, oxolinic acid, norfloxacin, perfloxacin, enoxacin, ofloxacin, ciprofloxacin, temafloxacin, lomefloxacin, fleroxacin, grepafloxacin, sparfloxacin, trovafloxacin, clinafloxacin, gatifloxacin, moxifloxacin, gemifloxacin, sitafloxacin, metronidazole, daptomycin, garenoxacin, ramoplanin, faropenem, polymyxin, tigecycline, AZD2563, and trimethoprim.

### Local anesthetics

Exemplary local anesthetics which can be used in the methods and compositions of the invention include, without limitation, cocaine, procaine, lidocaine, prilocaine, mepivicaine, bupivicaine, articaine, tetracaine, chloroprocaine, etidocaine, and ropavacaine.

### Antispasmodic

Exemplary antispasmodics which can be used in the methods and compositions of the invention include, without limitation, atropine, belladonna, bentyl, cystospaz, detrol (tolterodine), dicyclomine, ditropan, donnatol, donnazyme, fasudil, flexeril, glycopyrrolate, homatropine, hyoscyamine, levsin, levsinex, librax, malcotran, novartin, oxyphencyclimine, oxybutynin, pamine, tolterodine, tiquizium, prozapine, and pinaverium.

### Matrix Coating

The matrix coatings of the invention can be designed to vary in adhesion to a surface by varying the size of oligomers, their solubility in physiological media, and/or employing oligomers which favorably interact with the surface on which the coating is placed. Such favorable interactions can include, for example, coordinatation (i.e., carboxylate groups coordinating to a metal surface), and/or hydrogen bonding between the oligomers and the device surface. In certain embodiments, the matrix coating is applied to the surface of the implantable medical device to form a thin coating (i.e., 0.5-5.0 microns in thickness). Because the matrix coatings of the invention do not have the properties of a base polymer, they are not susceptible to flaking or cracking during the physical manipulation of the device, such as the crimping and deployment of a stent. The matrix coatings of the invention control the release of biologically active agents incorporated within the matrix by limiting the rate of diffusion of the agent from the matrix. The modified release profile is achieved despite the low molecular weight of the matrix component and the self-eliminating or bioerodible nature of the coating.

A primary function of such coating can be to locally deliver a biologically active agent for a defined period of time and leave the device surface intact once the therapy period is completed. The matrix coating is optionally complexed, or covalently tethered, or physically combined with a biologically active agent, or applied in a mixture including a biologically active agent. The amount of biologically active agent loaded into the matrix coating will depend upon the desired local concentration and release profile from the matrix coating.

The matrix coating of the invention is significantly different from biodegradable and bioabsorbable polymers as the oligomers of the matrix remain intact during elimination from the surface of the device. In certain embodiments, a biologically active agent is covalently bound to an oligomer in the matrix via a hydrolyzable linker. In these embodiments, it is understood that the hydrolysis of the linker can occur either within the matrix or after diffusion of the biologically active agent from the surface of the device. It is desirable to limit hydrolytic degradation near the site of implantation to avoid localized changes in pH and the generation of inflammatory side products.

The matrix coatings of the invention can be applied to the surface of a medical device in any number of ways including, but not limited, to dipping, spraying, brushing, printing, or spin coating of the matrix coating material from a solution or suspension followed by solvent removal step as needed. Further description of how the matrix coatings can be applied is found in the Examples.

### Coated Medical Devices

A wide variety of implantable medical devices can be coated using the compositions and methods of the invention. Implantable medical devices can be coated to improve their biocompatibility and to deliver biologically active agents at the site of implantation. The medical devices include, without limitation, catheters, guide wires, vascular stents, micro-particles, probes, sensors, drug depots, transdermal patches, vascular patches, and tubing. The medical device can be an implanted device, percutaneous device, or cutaneous device. Implanted devices include articles that are fully implanted in a patient, i.e., are completely internal. Percutaneous devices include items that penetrate the skin, thereby extending from outside the body into the body. Cutaneous devices are used superficially. Implanted devices include, without limitation, prostheses such as pacemakers, electrical leads such as pacing leads, defibrillators, artificial hearts, ventricular assist devices, anatomical reconstruction prostheses such as breast implants, artificial heart valves, heart valve stents, pericardial patches, surgical patches, coronary stents, vascular grafts, vascular and structural stents, vascular or cardiovascular shunts, biological conduits, pledges, sutures, annuloplasty rings, stents, staples, valved grafts, dermal grafts for wound healing, orthopedic spinal implants, orthopedic pins, intrauterine devices, urinary stents, maxial facial reconstruction plating, dental implants, intraocular lenses, clips, sternal wires, bone, skin, ligaments, tendons, and combination thereof. Percutaneous devices include, without limitation, catheters or various types, cannulas, drainage tubes such as chest tubes, surgical instruments such as forceps, retractors, needles, and catheter cuffs. Cutaneous devices include, without limitation, burn dressings, wound dressings and dental hardware, such as bridge supports and bracing components.

The following examples are put forth so as to provide those of ordinary skill in the art with a complete disclosure and description of how the methods and compounds claimed herein are performed, made, and evaluated, and are intended to be purely exemplary of the invention and are not intended to limit the scope of what the inventors regard as their invention.

### ACRONYMS

The following acronyms denote the listed compounds.
- ACD: acid citrate dextrose
- B AL: poly(difluoromethylene),α-fluoro-ω-(2-hydroxyethyl)
- BPH: neopentyl glycol phthalic anhydride based polyester diol
- CDCl₃: deuterated chloroform
- DBDL: dibutyltin dilaurate
- DCM: dichloromethane
- DIC: diisopropylcarbodiimide
- DL-PLGA: DL-polylactic-co-glycolic acid polymer
- DMAc: dimethylacetamide
- DMAP: 4-(dimethylamino)pyridine
- DMF: dimethylformamide
- DMSO: dimethylsulphoxide
- EtO: ethylene oxide
- HCAEC: human coronary artery endothelial cells
- HCl: hydrochloric acid
- HLB: hydroxy terminated polybutadiene
- HPCN: hexamethylene polycarbonate diol
- KBr: potassium bromide
- KD: dansyl labelled lysine
- LDI: lysine diisocyanate
- MeOH: methanol
- NaOH: sodium hydroxideExam
- N₂: nitrogen gas
- OPCN: methyl polycarbonate diol
- PBS: phosphate buffer solution
- PCAEC: porcine coronary artery endothelial cells
- PEB: polyethylene-co-butadiene diol
- PET: polyethylene terephthalate
- PCL: polycaprolactone
- PTT: partial thromboplastin time
- PTMO: polytetramethylene oxide
- PTX: paclitaxel
- RBC: red blood cell
- SA: salicylic acid
- TEA: triethylamine
- THF: tetrahydrofuran
- TMX: m-tetramethylxylene diisocyanate
- Tween PBS: 0.05% Tween 20 in phosphate buffer solution

### GENERAL EXPERIMENTAL PROTOCOLS

Cationic Solid Phase Extraction (SCX-SPE): A pre-packed cationic silica gel column (plastic) is used to remove small cationic compounds from the reaction mixtures.
Fluorous Solid Phase Extraction (F-SPE): SPE substrates modified with perfluorinated ligands (F-SPE) are used to selectively retain perfluorinated oligomers, allowing the separation of non-fluorinated compounds.
Elemental analysis: samples are combusted, and the liberated fluorine is absorbed into water and analyzed by ion-selective electrode.
FTIR analysis: a sample is dissolved as a 20 mg/mL solution in a suitable volatile solvent and 50 µL of this solution is cast on a KBr disk. Once dried, the sample is analyzed.
GPC analysis: samples are dissolved as a 20 mg/mL solution in a suitable solvent (THF, dioxane, DMF) and are analyzed using a polystyrene column calibrated with polystyrene standards.
NMR: samples are dissolved at 20 mg/mL in a suitable solvent and are analyzed using a 300 or 400 MHz NMR spectrometer.
SEM: surfaces are coated with gold and images taken wih an accelerating volatage of 20 kV.
XPS analysis: films are analyzed using a 90° take-off angle.
Oligomers: fluorinated and non fluorinated oligomers of different chemical compositions are made to evaluate their coating, residency time and compatibility with pharmaceutical compounds.
Pharmaceuticals: these compounds are selected according to mode of cellular interactions and functional groups available for interactions with drug delivery matrix.
Coating: coating methods are developed to demonstrate and establish thin coating of oligomers.
Residency time: flow and sink conditions (artificial urine, porcine blood, bovine blood, porcine plasma and cardiac muscle) are used to measure residency time

### EXAMPLE 1: Synthesis and characterization of Compound 1 (oligofluoro-ester).

PTMO (15.0 g, 14 mmol) was reacted with LDI (5.9 g, 28 mmol) in DMAc (80 mL) in the presence of DBDL catalyst, at 70°C for two hours under N₂. Perfluoroalcohol (13.15 g, 31 mmol) was dissolved in DMAc (25 mL), added to the reaction, and stirred at room temperature overnight under N₂. The product (Compound 1) was purified by solvent extraction and cationic SPE. GPC (dioxane mobile phase): retention time of 25 minutes. ¹H NMR (400 MHz, CDCl₃) δ (ppm) 4.24-4.46 (-CH₂-O, BAL), 3.94-4.13 (-CH₂-O-CO, PTMO), 3.74 (CH₃, LDI), 3.28-3.50 (CH₂-O, PTMO), 2.98-3.28 (CH₂-NH, LDI), 2.29-2.60 (-CH₂-CF₂-, BAL), 1.16-1.96 (PTMO and LDI CH₂). IR analysis was in accordance with the chemical structure: 3318 cm⁻¹ v(N-H) H-bonded, 2930 cm⁻¹ ν(C-H), 2848 cm⁻¹ ν(C-H), 1712 cm⁻¹ ν(C=O) urethane amide, 1524 cm⁻¹ ν(C-N), 1438 cm⁻¹ ν(C-N), 1356 cm⁻¹ ν(C-O), 1400-1000 cm⁻¹ ν(C-F). Elemental analysis: 20% F. DSC analysis: T_{g} = -69°C. Compound 1 was further purified by dissolving in MeOH and dialyzing for three days using 1000 MWCO regenerated cellulose membranes (Compound 1-D).

### EXAMPLE 2: Synthesis and characterization of Compound 2 (oligofluoro-acid).

Compound 1 was dissolved in MeOH and treated with 1N NaOH. The product (Compound 2) was neutralized with 1N HCl, precipitated in water, and dried. GPC (dioxane mobile phase): retention time of 25 minutes. ¹H NMR (400 MHz, CDCl₃) δ (ppm) 4.26-4.48 (-CH₂-O, BAL), 3.96-4.23 (-CH₂-O-CO, PTMO), 3.30-3.52 (CH₂-O, PTMO), 3.07-3.22 (CH₂-NH, LDI), 2.36-2.55 (-CH₂-CF₂-, BAL), 1.14-1.94 (PTMO and LDI CH₂). IR analysis was in accordance with the chemical structure: 3318 cm⁻¹ ν(N-H) H-bonded, 2930 cm⁻¹ ν(C-H), 2848 cm⁻¹ v(C-H), 1712 cm⁻¹ ν(C=O) urethane amide, 1524 cm⁻¹ ν(C-N), 1438 cm⁻¹ v(C-N), 1356 cm⁻¹ v(C-O), 1400-1000 cm⁻¹ ν(C-F). Compound 2 was further purified by dissolving in MeOH and dialyzing for three days using 1000 MWCO regenerated cellulose membranes (Compound 2-D).

### EXAMPLE 3: Synthesis and characterization of Compound 3 (oligofluoro-dansyl, covalent conjugation).

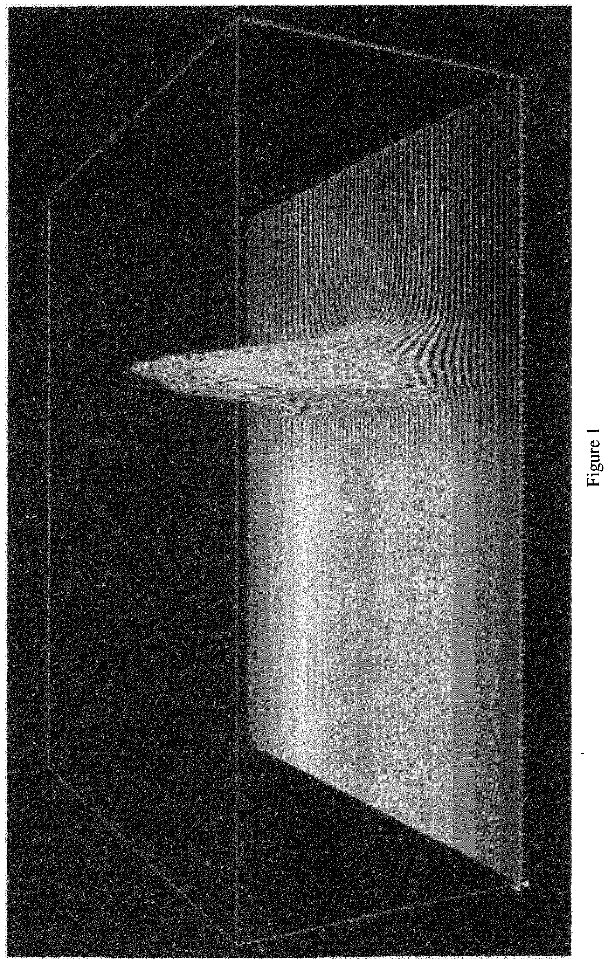

Compound 2-D (2.0 g, 1.71 mmol acid) was dissolved in anhydrous DMF (25 mL). The solution was chilled, DIC (0.215 g, 1.71 mmol) was added and the solution was stirred for 2 hours at room temperature under N₂. TEA (0.345 g, 3.41 mmol) and dansyl-labelled lysine (KD) (0.718 g, 1.71 mmol) in anhydrous DMF (9 mL) were added to the activated Compound 2-D, and the solution was kept well stirred for 12 hours at room temperature under N₂. The product (Compound 3) was purified with cationic and fluorous SPE, and recovered by rotary evaporation. GPC (dioxane mobile phase): no free KD was detected, and the polymer peak had strong UV absorbance. ¹H NMR (400 MHz, CDCl₃) δ (ppm) 7.14-8.59 (aromatic H, KD) 4.46-4.66 (CH₂-N, KD), 4.28-4.48 (-CH₂-O, BAL), 3.90- 4.17 (-CH₂-O-CO, PTMO), 3.31-3.54 (CH₂-O, PTMO), 3.06-3.26 (CH₂-NH, LDI), 2.81-3.00 (CH₃, KD) 2.32-2.58 (-CH₂-CF₂-, BAL), 1.08-1.94 (CH₂, PTMO, LDI and KD). High performance liquid chromatography (HPLC) analysis of Compound 3: samples ranging in concentration from 0.0005 to 50 mg/mL in MeOH were injected and analyzed using an MeOH/pH 9 buffer mobile phase. Free KD (standard solution) eluted at 21 minutes, and Compound 3 eluted at 35 minutes with no evidence of free KD contamination (Figure 1).

### EXAMPLE 4: Synthesis and characterization of Compound 4 (oligofluoro-PTX, covalent conjugation).

Compound 2-D (1.5 g, 1.36 mmol acid) was dissolved in anhydrous DCM (150 mL). The solution was chilled, DIC (0.342 g, 2.71 mmol) was added and the solution was stirred for 2 hours at room temperature under N₂. DMAP (0.496 g, 4.07 mmol) and PTX (2.31 g, 2.71 mmol) in anhydrous DCM (75 mL) were added to the activated Compound 2-D, and the solution was kept well stirred for three days at room temperature under N₂. The product (Compound 4) was purified with fluorous SPE, and recovered by rotary evaporation. GPC (dioxane mobile phase): no free PTX was detected, and the polymer peak had a strong UV absorbance. ¹H NMR (300 MHz, DMSO) δ (ppm) 7.11-8.15 (aromatic H, PTX), 6.24-6.30 (C10), 5.79-5.88 (C3'), 5.53-5.63 (C2' conjugated), 5.32-5.44 (C2), 4.81-4.93 (C5), 4.56-4.62 (C7), 4.16-4.35 (-CH₂-O, BAL), 3.84-3.96 (-CH₂-O-CO, PTMO), 3.13-3.40 (CH₂-O, PTMO), 2.21-2.27 (OAc, C4), 2.02-2.09 (OAc, C10), 1.68-1.70 (C18), 1.04-1.60 (CH₂, PTMO, LDI), 0.90-1.02 (C16, C17).

### EXAMPLE 5: Synthesis and chracterization of Compound 5 (oligomer-MeOH).

PTMO (15.0 g, 14 mmol) was reacted with LDI (5.9 g, 28 mmol) in DMAc (60 mL) in the presence of DBDL catalyst, at 70°C for two hours under N₂. MeOH (0.9 g, 28 mmol) was dissolved in DMAc (25 mL), added to the reaction, and stirred at room temperature overnight under N₂. The product (Compound 5) was purified by solvent extraction and cationic SPE. GPC (dioxane mobile phase): 26 minutes. ¹H NMR (300 MHz, CDCl₃) δ (ppm) 4.27-4.39 (-CH-, LDI), 4.02-4.14 (-CH₂-O-CO, PTMO), 3.73-3.78 (CH₃, LDI), 3.60-3.70 (-OCH₃, MeOH), 3.30-3.53 (CH₂-O, PTMO), 3.09-3.21 (CH₂-NH, LDI), 1.22-1.91 (PTMO and LDI CH₂).

### EXAMPLE 6: Synthesis and characterization of Compound 6 (TMX-oligofluoro).

PTMO (15.0 g, 14 mmol) was reacted with TMX (6.79 g, 28 mmol) in DMAc (60 mL) in the presence of DBDL catalyst, at 70°C for two hours under N₂. Perfluoroalcohol (12.85 g, 30 mmol) was dissolved in DMAc (20 mL), added to the reaction, and stirred at room temperature overnight under N₂. The product (Compound 6) was purified by solvent extraction. GPC (dioxane mobile phase): retention time of 26.5 minutes. ¹H NMR (300 MHz, CDCl₃) δ (ppm) 7.2-7.4 (-CH-, TMX), 4.23-4.37 (-CH₂-O-, BAL), 3.9-4.06 (-CH₂-O-CO, PTMO), 3.32- 3.52 (CH₂-O, PTMO), 2.29-2.57 (-CH₂-CF₂-, BAL), 1.16-1.96 (-CH₂ -PTMO and -CH₃ TMX). IR analysis was in accordance with the chemical structure: 3308 cm⁻¹ v(N-H) H-bonded, 2936 cm⁻¹ v(C-H), 2852 cm⁻¹ v(C-H), 1716 cm⁻¹ ν(C=O) urethane amide, 1520 cm⁻¹ v(C-N), 1456 cm⁻¹ v(C-N), 1362 cm⁻¹ v(CO), 1400-1000 cm⁻¹ v(C-F). DSC analysis: Tg = -55°C. Compound 6 was further purified by dissolving in MeOH and dialyzing for three days using 1000 MWCO regenerated cellulose membranes (Compound 6-D).

### EXAMPLE 7: Synthesis and characterization of Compound 7 (PCL-oligofluoro).

PCL diol (10 g, 8 mmol) was reacted with LDI (3.39 g, 16 mmol) in DMAc (17 mL) in the presence of DBDL catalyst, at 70°C for two hours under N₂. Perfluoroalcohol (7.39 g, 18 mmol) was dissolved in DMAc (20 mL), added to the reaction, and stirred at room temperature overnight under N₂. The product (Compound 7) was purified by solvent extraction and cationic SPE. GPC (dioxane mobile phase): retention time of 26.8 minutes, no free PCL diol detected. ¹H NMR (400 MHz, CDCl₃) δ (ppm) 4.27- 4.48 (-CH₂-O, BAL), 4.17- 4.26 (-CH₂-O-CO-N, PCL), 3.96-4.12 (-CH2-O-CO-, PCL), 3.71-3.76 (CH₃, LDI), 3.09-3.22 (CH₂-NH, LDI), 2.39- 2.53 (-CH₂-CF₂-, BAL), 2.26-2.38 (CO-CH₂-, PCL), 1.13-1.76 (PCL and LDI CH₂). DSC analysis: T_{g} = -53°C, Tₘ = 39°C. Compound 7 was further purified by dissolving in acetone and dialyzing for three days using 1000 MWCO regenerated cellulose membranes (Compound 7-D).

### EXAMPLE 8: Synthesis and characterization of Compound 8 (OPCN-oligofluoro).

Methyl polycarbonate diol (OPCN, 10.0 g, 10 mmol) was reacted with LDI (4.24 g, 20 mmol) in anhydrous DMAc (70 mL) in the presence of DBDL catalyst, at 70°C for two hours under N₂. Perfluoroalcohol (9.24 g, 22 mmol) was dissolved in anhydrous DMAc (25 mL), added to the reaction, and stirred at room temperature overnight under N₂. The product (Compound 8) was purified by solvent extraction and cationic SPE. GPC (dioxane mobile phase): retention time of 24.7 minutes. ¹H NMR (400 MHz, CDCl₃) δ (ppm) 4.27-4.51 (-CH₂-O, BAL), 3.82-4.07 (-CH₂-O, OPCN), 3.65-3.80 (-CH₃, LDI), 3.07-3.27 (CH₂-NH, LDI), 2.32-2.59 (-CH₂-CF₂-, BAL), 1.18-1.94 (CH₂,LDI), 0.84-1.09 (-CH₃, OPCN). DSC analysis: T_{g} = -3°C. Compound 8 was further purified by dissolving in acetone and dialyzing for three days using 1000 MWCO regenerated cellulose membranes (Compound 8-D).

### EXAMPLE 9: Synthesis and characterization of Compound 9 (HPCN-oligofluoro).

Hexamethylene polycarbonate diol (HPCN, 10.0 g, 5 mmol) was reacted with LDI (2.12 g, 10 mmol) in anhydrous DMAc (65 mL) in the presence of DBDL catalyst, at 70°C for two hours under N₂. Perfluoroalcohol (4.62 g, 11 mmol) was dissolved in anhydrous DMAc (15 mL), added to the reaction, and stirred at room temperature overnight under N₂. The product (Compound 9) was purified by solvent extraction and cationic SPE. GPC (dioxane mobile phase): retention time of 24.2 minutes. ¹H NMR (400 MHz, CDCl₃) δ (ppm) 4.30-4.43 (-CH₂-O, BAL), 3.97-4.22 (-CH₂-O, HPCN), 3.69-3.78 (-CH₃, LDI), 3.10-3.23 (CH₂-NH, LDI), 2.37-2.55 (-CH₂-CF₂-, BAL), 1.13-1.89 (CH₂, LDI and HPCN). DSC analysis: T_{g} = -40°C. Compound 9 was further purified by dissolving in acetone and dialyzing for three days using 1000 MWCO regenerated cellulose membranes (Compound 9-D).

### EXAMPLE 10: Synthesis and characterization of Compound 10 (PEB LDI-oligofluoro).

PEB diol (14.96 g, 6.0 mmol) was reacted with LDI (2.54 g, 12.0 mmol) in anhydrous toluene (60 mL) in the presence of DBDL catalyst, at 70°C for two hours under N₂. Perfluoroalcohol (5.541 g, 13.2 mmol) was dissolved in anhydrous toluene (20 mL) with slight heating, added to the reaction, and stirred at 70°C overnight under N₂. The product (Compound 10) was purified by solvent extraction and cationic SPE. GPC (THF mobile phase): retention time of 21 minutes. ¹H NMR (300 MHz, CDCl₃) δ (ppm) 4.29-4.45 (CH₂-O, BAL), 4.02-4.11 (-CH-, LDI, -CH-O-CO, PEB), 3.92-4.02 (-CH₂-O-CO, PEB), 3.75 (-CH₃, LDI), 3.10-3.22 (-CH₂-NH-, LDI), 2.38-2.57 (-CH₂-CF₂-, BAL), 0.76-1.92 (-CH₂ PEB and LDI, -CH₃, -CH- PEB). DSC analysis: T_{g} = -16°C.

### EXAMPLE 11: Synthesis and characterization of Compound 11 (PEB TMX-oligofluoro).

PEB diol (15.10 g, 6.0 mmol) was reacted with TMX (2.94 g, 12.1 mmol) in anhydrous toluene (60 mL) in the presence of DBDL catalyst, at 70°C for two hours under N₂. Perfluoroalcohol (5.55 g, 13.2 mmol) was dissolved in anhydrous toluene (20 mL) with slight heating, added to the reaction, and stirred at 70°C overnight under N₂. The product (Compound 11) was purified by solvent extraction and cationic SPE. GPC (THF mobile phase): retention time of 21.5 minutes. ¹H NMR (300 MHz, CDCl₃) δ (ppm) 7.20-7.48 (-CH-, TMX), 4.22-4.37 (-CH₂-O, BAL), 3.90-4.03 (-CH-O-CO, PEB), 3.85-3.92 (-CH₂-O-CO, PEB), 2.33-2.55 (-CH₂-CF₂-, BAL), 0.71-1.72 (-CH₂-, CH₃, -CH-, PEB and -CH₃, TMX). DSC analysis: T_{g} = -13°C.

### EXAMPLE 12: Synthesis and characterization of Compound 12 (HLB-oligofluoro).

LBH-P hydrogenated hydroxyl terminated polybutadiene (HLB, 10.0 g, 5 mmol) was reacted with LDI (2.12 g, 10 mmol) in anhydrous toluene (65 mL) in the presence of DBDL catalyst, at 70°C for two hours under N₂. Perfluoroalcohol (4.62 g, 11 mmol) was dissolved in anhydrous toluene (15 mL), brought to 45°C, added to the reaction, and stirred at room temperature overnight under N₂. The product (Compound 12) was purified by solvent extraction and cationic SPE. GPC (dioxane mobile phase): retention time of 23.9 minutes. ¹H NMR (400 MHz, CDCl₃) δ (ppm) 4.28-4.46 (-CH₂-O, BAL), 4.00-4.14 (-CH₂-O, HLB), 3.72-3.80 (-CH₃, LDI), 3.08-3.22 (CH₂-NH, LDI), 2.37-2.54 (-CH₂-CF₂-, BAL), 0.57-1.75 (CH₂ and CH, LDI and HLB). DSC analysis: Tg = -49°C.

### EXAMPLE 13: Synthesis and characterization of Compound 13 (BPH-oligofluoro).

Neopentyl glycol phthalic anhydride based polyester diol (BPH, 10.0 g, 10 mmol) was reacted with LDI (4.24 g, 20 mmol) in anhydrous DMAc (70 mL) in the presence of DBDL catalyst, at 70°C for two hours under N₂. Perfluoroalcohol (9.24 g, 22 mmol) was dissolved in anhydrous DMAc (25 mL), added to the reaction, and stirred at room temperature overnight under N₂. The product (Compound 13) was purified by solvent extraction and cationic SPE. GPC (dioxane mobile phase): retention time of 25.4 minutes. ¹H NMR (400 MHz, CDCl₃) δ (ppm) 7.41-7.79 (aromatic H, BPH), 4.25-4.44 (-CH₂-O, BAL), 4.05-4.21 (-CH₂-O, BPH), 3.67-3.79 (-CH₃, LDI), 3.06-3.25 (CH₂-NH, LDI), 2.32-2.56 (-CH₂-CF₂-, BAL), 1.26-1.90 (CH₂, LDI), 0.86-1.11 (-CH₃, BPH).

### EXAMPLE 14: Synthesis and characterization of Compound 14 (322-PT-oligofluoro).

PTMO (5.0 g, 5 mmol) was reacted with LDI (1.59 g, 7.5 mmol) in anhydrous DMAc (35 mL) in the presence of DBDL catalyst, at 70°C for two hours under N₂. Perfluoroalcohol (2.31 g, 5.5 mmol) was dissolved in anhydrous DMAc (10 mL), added to the reaction, and stirred at room temperature overnight under N₂. The product (Compound 14) was purified by solvent extraction and cationic SPE. GPC (dioxane mobile phase): retention time of 24 minutes. ¹H NMR (400 MHz, CDCl₃) δ (ppm) 4.23-4.49 (-CH₂-O, BAL), 4.00-4.18 (-CH₂-O, PTMO), 3.69-3.79 (-CH₃, LDI), 3.30-3.59, - CH₂-O, PTMO), 3.09-3.25 (CH₂-NH, LDI), 2.37-2.57 (-CH₂-CF₂-, BAL), 1.09-1.94 (CH₂, LDI and PTMO). Elemental analysis: 13.7 wt% F.

### EXAMPLE 15: Synthesis and characterization of Compound 15 (652-PT-oligofluoro).

PTMO (5.0 g, 5 mmol) was reacted with LDI (1.27 g, 6 mmol) in anhydrous DMAc (35 mL) in the presence of DBDL catalyst, at 70°C for two hours under N₂. Perfluoroalcohol (0.92 g, 2.2 mmol) was dissolved in anhydrous DMAc (5 mL), added to the reaction, and stirred at room temperature overnight under N₂. The product (Compound 15) was purified by solvent extraction and cationic SPE. GPC (dioxane mobile phase): retention time of 23 minutes. ¹H NMR (400 MHz, CDCl₃) δ (ppm) 4.27-4.44 (-CH₂-O, BAL), 3.98-4.17 (-CH₂-O, PTMO), 3.69-3.79 (-CH₃, LDI), 3.27-3.52, - CH₂-O, PTMO), 3.09-3.22 (CH₂-NH, LDI), 2.34-2.54 (-CH₂-CF₂-, BAL), 1.01-1.90 (CH₂, LDI and PTMO). Elemental analysis: 4.3 wt% F.

### EXAMPLE 16: Synthesis and characterization of Compound 16 (12112-PT-oligofluoro).

PTMO (10.0 g, 10 mmol) was reacted with LDI (2.32 g, 10.9 mmol) in anhydrous DMAc (115 mL) in the presence of DBDL catalyst, at 70°C for two hours under N₂. Perfluoroalcohol (0.84 g, 2 mmol) was dissolved in anhydrous DMAc (5 mL), added to the reaction, and stirred at room temperature overnight under N₂. The product (Compound 16) was purified by solvent extraction and cationic SPE. GPC (dioxane mobile phase): retention time of 22 minutes. ¹H NMR (400 MHz, CDCl₃) δ (ppm) 4.27-4.44 (CH₂-O, BAL), 3.98-4.17 (-CH₂-O, PTMO), 3.69-3.79 (-CH₃, LDI), 3.27-3.52 (-CH₂-O, PTMO), 3.09-3.22 (CH₂-NH, LDI), 2.34-2.54 (-CH₂-CF₂-, BAL), 1.01-1.90 (CH₂, LDI and PTMO). Elemental analysis: 1.3 wt% F.

### EXAMPLE 17: Coating of Compound 1 on a stent, with evaluation of coating pre and post deployment in PBS.

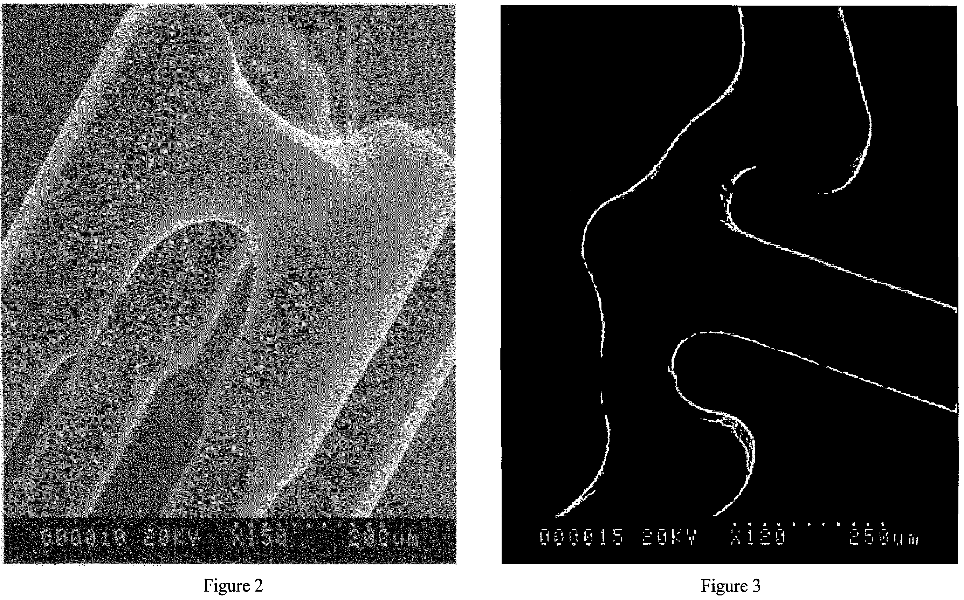

Compound 1 (1.0 g) was dissolved in toluene, stirred for 24 hours at room temperature and kept at room temperature until use. The solution was sprayed onto stents using an EFD spray system with settings specific to Compound 1, and the stents were placed in a 50°C flow oven for 20-24 hours for drying. SEM analysis (Figure 2) indicated a thin coating with minimum webbing between struts. In addition, Compound 1 coating was evaluated after crimping the stent on a balloon and deploying under PBS in 0.125"ID x 0.25"OD Sil-Tec medical grade silicone tubing at 37°C (Figure 3).

### EXAMPLE 18: Coating of Compound 2 on a stent.

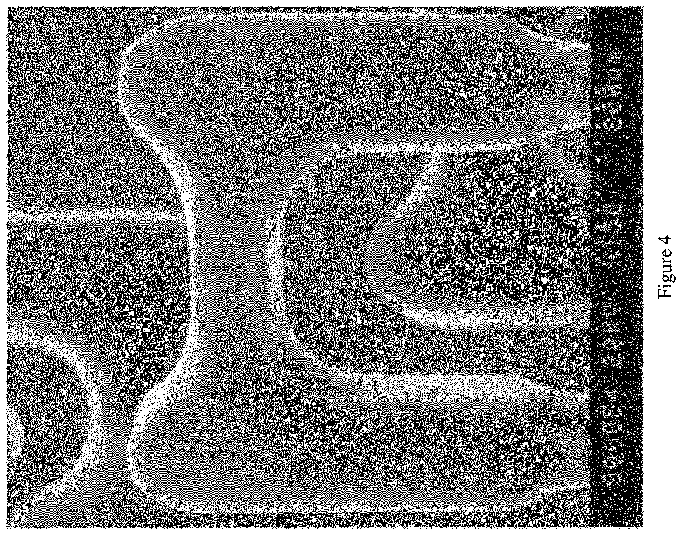

Compound 2 (1.0 g) was dissolved in THF:toluene, stirred for 24 hours at room temperature and kept at room temperature until use. The solution was sprayed onto stents using an EFD spray system with settings specific to Compound 2, and the stents were placed in a 50°C flow oven for 20-24 hours for drying. SEM analysis (Figure 4) suggested a thin coating with minimal webbing between struts.

### EXAMPLE 19: Coating of Compound 3 on a stent, with evaluation of coating pre and post deployment in air.

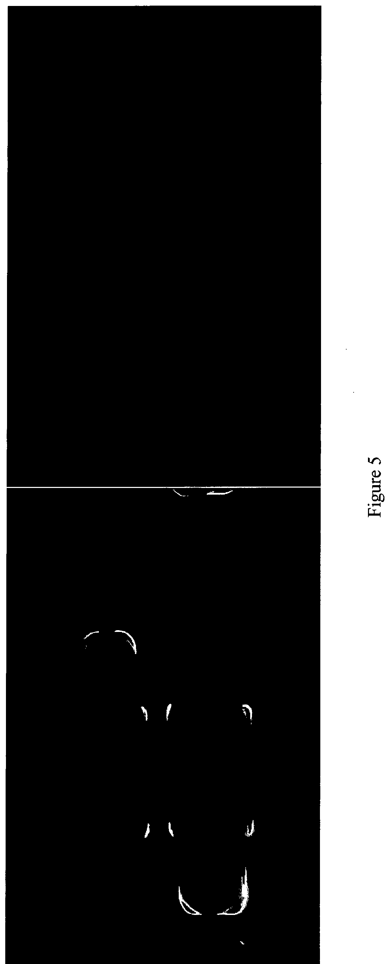

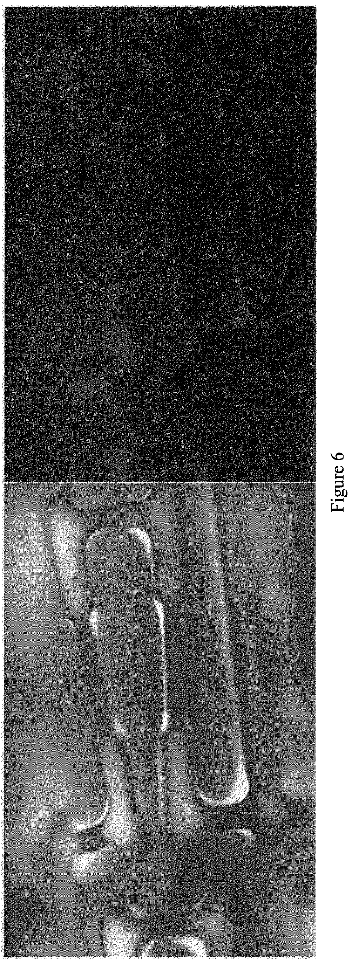

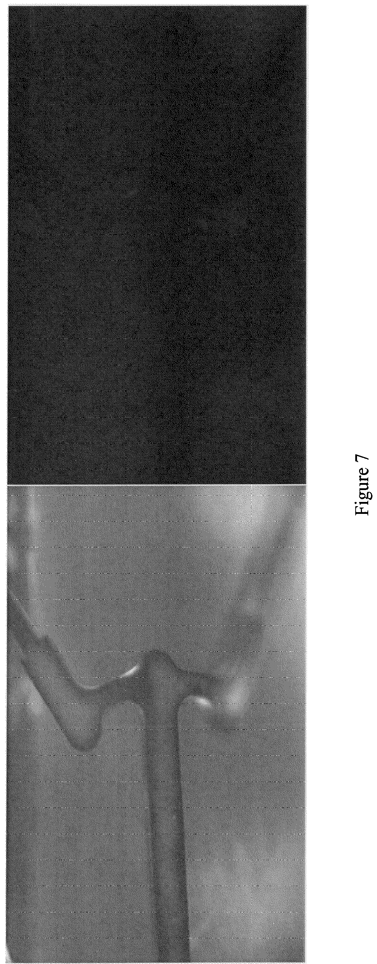

Compound 3 (0.2 g) was dissolved in THF:toluene, stirred for 24 hours at room temperature, and used immediately. The solution was sprayed onto stents using an EFD spray system with settings specific to Compound 3, and the stents were placed in a 50°C flow oven for 20-24 hours for drying. Confocal / fluorescence microscopy images were taken and indicated the presence of coating on the stents (Figure 5). In addition, Compound 3 coating had sufficient integrity during product processing, including crimping on a balloon (Figure 6) and deployment in air (Figure 7).

### EXAMPLE 20: Coating of Compound 4 on a stent and release of PTX into Tween PBS.

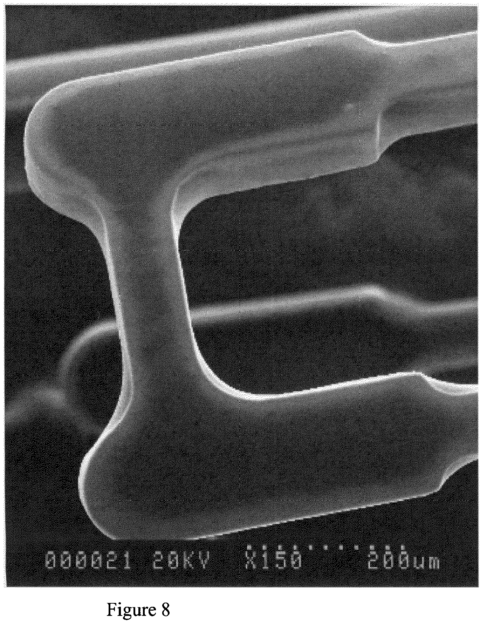

Compound 4 (0.2 g) was dissolved in toluene and stirred for 24 hours at room temperature. The solution was sprayed onto stents using an EFD spray system with settings specific to Compound 4, and the stents were dried in a 50°C flow oven for 20-24 hours. SEM analysis (Figure 8) indicated a thin coating with minimum webbing between struts. PTX release was investigated at 37°C with Tween PBS. Tween PBS (1 mL) was added to each stent and changed daily. PTX release was measured using RP-HPLC with benzonitrile as the internal standard: (ng/ml): (day 1) = 32, (day 2) = 25, (day 3) = 22, (day 4) = 6, (day 5) = 3, (day 6) = 2.

### EXAMPLE 21: Coating of Compound 5 on a stent.

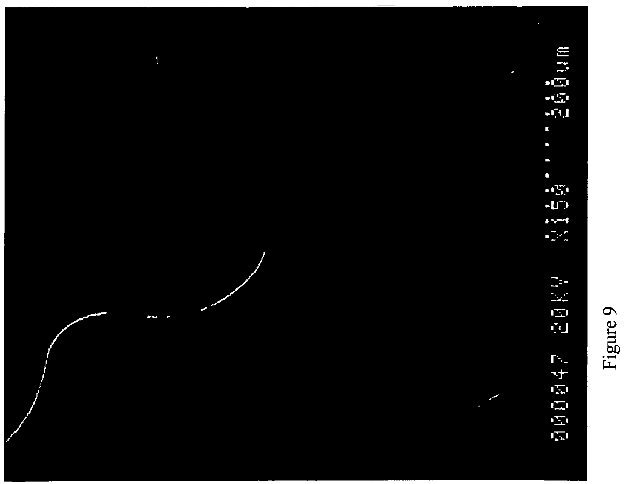

Compound 5 (0.2 g) was dissolved in toluene, stirred for 24 hours at room temperature, and stored at room temperature until use. The solution was sprayed onto stents using an EFD spray system with settings specific to Compound 5, and the stents were placed in a 50°C flow oven for 20-24 hours for drying. SEM images (Figure 9) suggested a thin coating with minimum webbing between struts.

### EXAMPLE 22: Coating of Compound 6 on a stent.

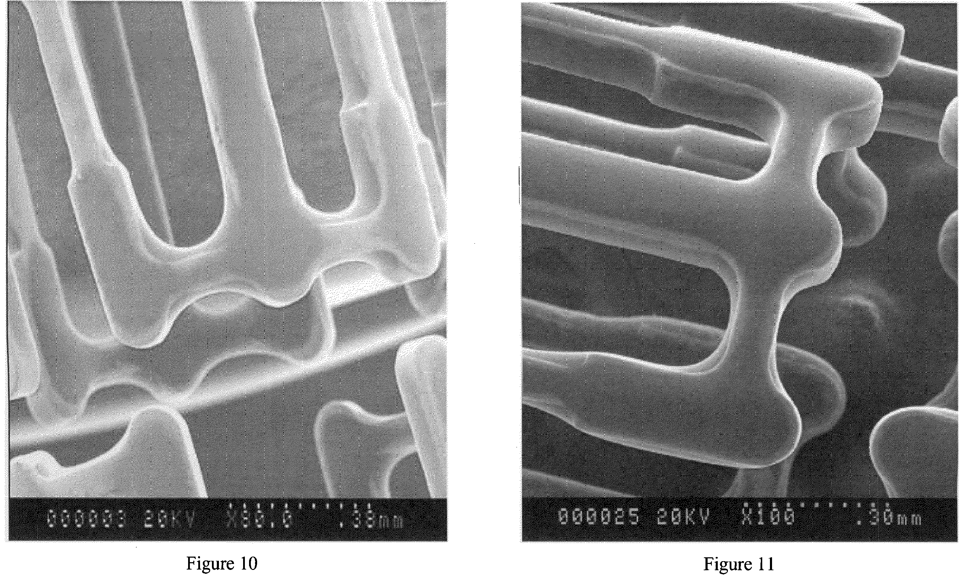

Compound 6 (0.2 g) was dissolved in toluene, stirred for 24 hours at room temperature, and stored at room temperature until use. The solution was sprayed onto stents using an EFD spray system with settings specific to Compound 6, and the stents were placed in a 50°C flow oven for 20-24 hours for drying. SEM images (Figure 10) indicated a thin coating with minimum webbing between struts.

### EXAMPLE 23: Coating of Compound 7 on a stent.

Compound 7 (0.2 g) was dissolved in toluene, and stirred for 24 hours at room temperature until used for coating. The solution was sprayed onto stents using an EFD spray system with settings specific to Compound 7. The stents were dried in a 50°C flow oven for 20-24 hours. SEM image collection was used to validate the coating quality (Figure 11).

### EXAMPLE 24: Coating of Compound 8 on a stent.

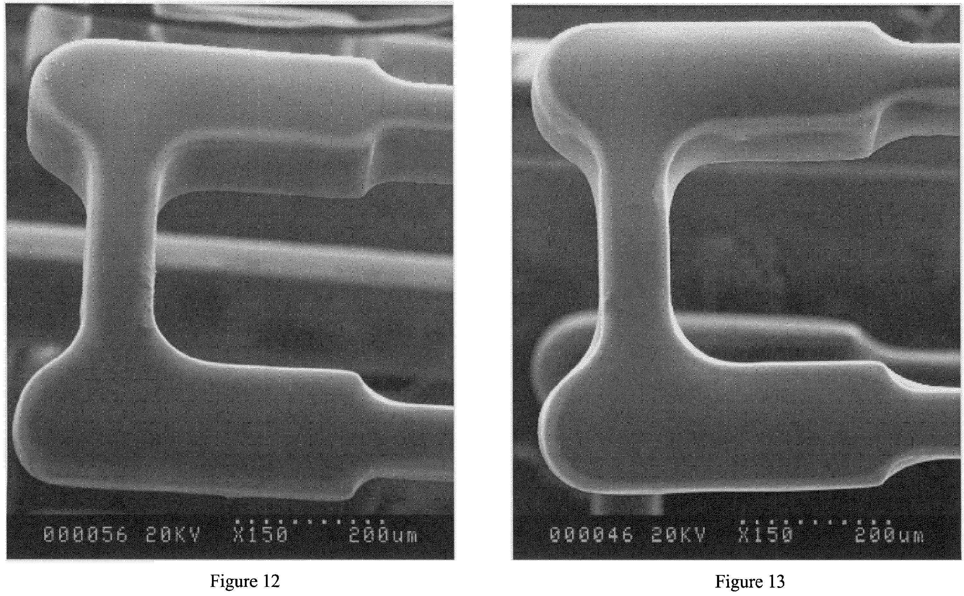

Compound 8 (0.4 g) was dissolved in THF, stirred for 24 hours at room temperature and used for coating. The solution was sprayed onto stents using an EFD spray system with settings specific to Compound 8. The stents were dried at room temperature in a fume hood for 24 hours. SEM images (Figure 12) indicated a thin coating with minimum webbing between struts.

### EXAMPLE 25: Coating of Compound 9 on a stent.

Compound 9 (0.4 g) was dissolved in THF, stirred for 24 hours at room temperature and used for coating. The solution was sprayed onto stents using an EFD spray system with settings specific to Compound 9. The stents were dried at room temperature in a fume hood for 24 hours. SEM images (Figure 13) indicated a thin coating with minimum webbing between struts.

### EXAMPLE 26: Coating of Compound 10 on a stent.

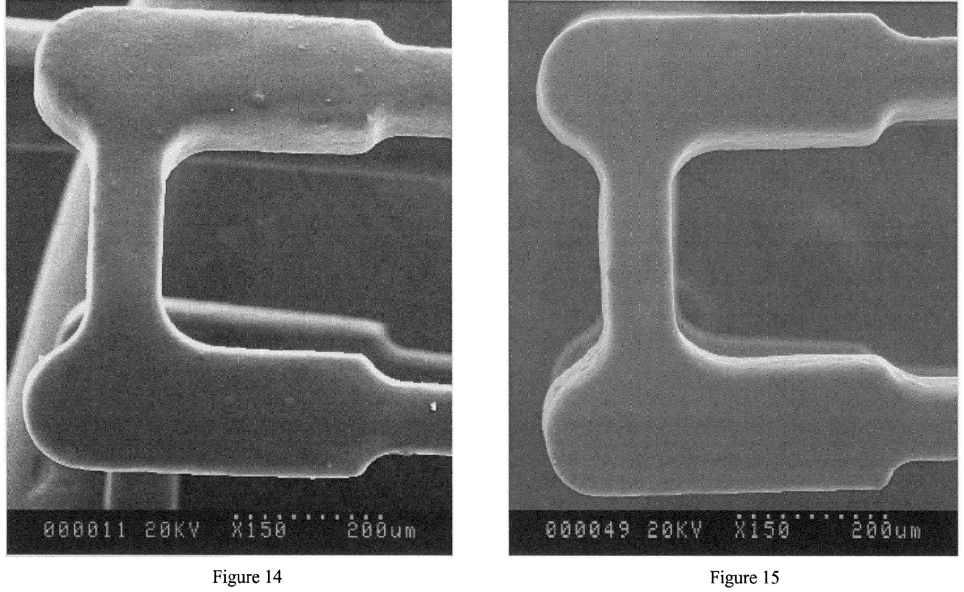

Compound 10 (0.2 g) was dissolved in toluene, stirred for 24 hours at room temperature and used for coating. The solution was sprayed onto stents using an EFD spray system with settings specific to Compound 10. The stents were dried in a 50°C flow oven for 20-24 hours. SEM images (Figure 14) indicated a thin coating with minimum webbing between struts.

### EXAMPLE 27: Coating of Compound 11 on a stent.

Compound 11 (0.2 g) was dissolved in toluene, stirred for 24 hours at room temperature and used for coating. The solution was sprayed onto stents using an EFD spray system with settings specific to Compound 11. The stents were dried in a 50°C flow oven for 20-24 hours. SEM images (Figure 15) indicated a thin coating with minimum webbing between struts.

### EXAMPLE 28: Coating of Compound 12 on a stent.

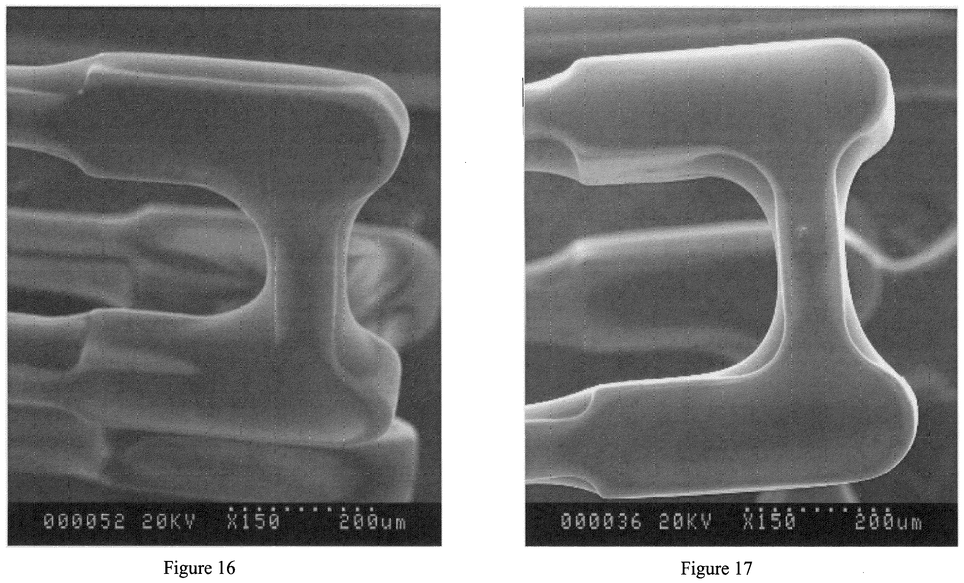

Compound 12 (0.4 g) was dissolved in chloroform, stirred for 24 hours at room temperature and used for coating. The solution was sprayed onto stents using an EFD spray system with settings specific to Compound 12. The stents were dried at room temperature in a fume hood for 3 days. SEM images (Figure 16) indicated a thin coating with minimum webbing between struts.

### EXAMPLE 29: Coating of Compound 14 on a stent.

Compound 14 (0.4 g) was dissolved in THF, stirred for 24 hours at room temperature and used for coating. The solution was sprayed onto stents using an EFD spray system with settings specific to Compound 14. The stents were dried at room temperature in a fume hood for 3 days. SEM images (Figure 17) indicated a thin coating with minimum webbing between struts.

### EXAMPLE 30: Coating of Compound 15 on a stent.

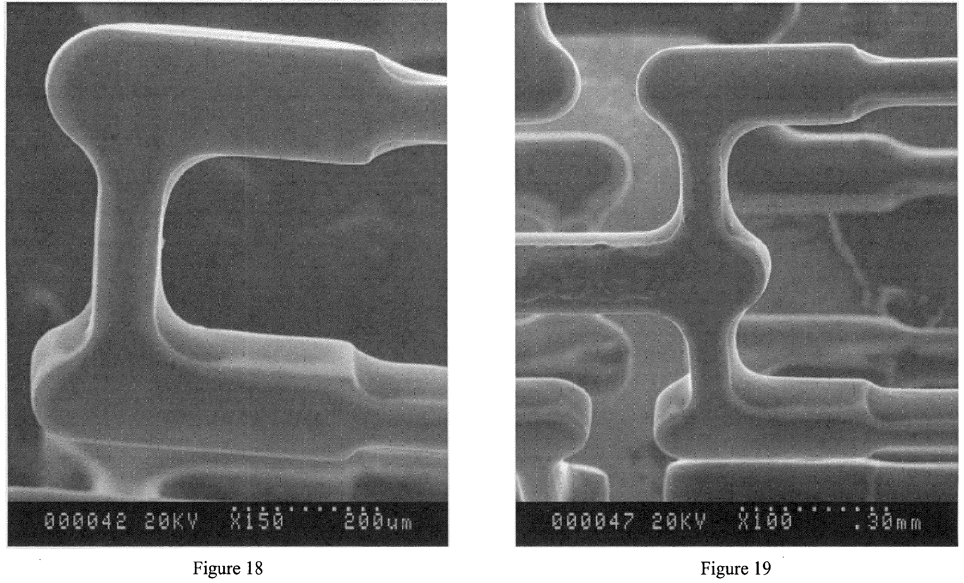

Compound 15 (0.4 g) was dissolved in THF, stirred for 24 hours at room temperature and used for coating. The solution was sprayed onto stents using an EFD spray system with settings specific to Compound 15. The stents were dried at room temperature in a fume hood for 3 days. SEM images (Figure 18) indicated a thin coating with minimum webbing between struts.

### EXAMPLE 31: Coating of Compound 1 + Compound 7 on a stent.

Compound 1 (0.2 g) was dissolved in THF and added to Compound 7 (0.2 g) that was dissolved in THF. The resulting solution was stirred for 24 hours at room temperature and used for coating. The solution was sprayed onto stents using an EFD spray system with settings specific to Compound 1+7. The stents were dried at room temperature in a fume hood for 3 days. SEM images (Figure 19) indicated a thin coating with minimum webbing between struts.

### EXAMPLE 32: Preparation of Compound 2 + 8.8 wt% PTX, and coating on a stent.

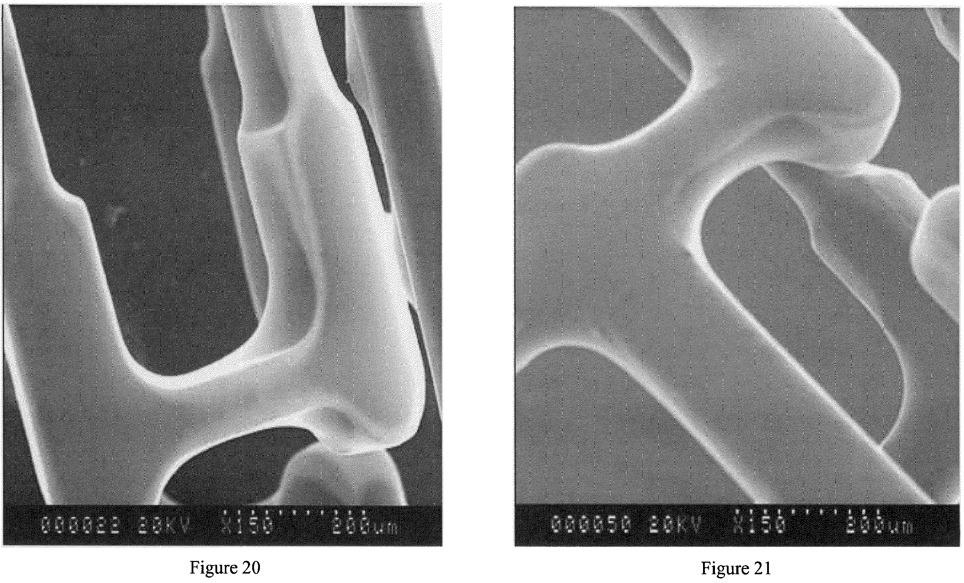

Compound 2 (0.332 g) and PTX (0.032 g) were dissolved in THF:toluene, stirred for 24 hours at room temperature, and used immediately. The solution was sprayed onto stents using an EFD spray system with settings specific to Compound 2 + PTX, and the stents were placed in a 50°C flow oven for 20-24 hours for drying. SEM analysis (Figure 20) indicated a thin coating with minimum webbing between struts.

### EXAMPLE 33: Preparation of Compound 6 + 8.8 wt% PTX, and coating on a stent.

Compound 6 (0.332 g) and PTX (0.032 g) were dissolved in THF:toluene, was stirred for 24 hours at room temperature, and used immediately. The solution was sprayed onto stents using an EFD spray system with settings specific to Compound 6 + 8.8 wt% PTX, and the stents were placed in a 50°C flow oven for 20-24 hours for drying. SEM analysis (Figure 21) indicated a thin coating with minimum webbing between struts.

### EXAMPLE 34: Combination of Compounds 1, 2, 5, 6 and 7 with salicylic acid (SA) and the release profile of SA.

Compounds 1, 2, 5, 6 and 7 (0.075 g) were mixed with SA (0.025 g) in MeOH under nitrogen protection, stirred, and were separated from solvent by rotary evaporation and vacuum drying. Controls were also prepared: Compounds 1, 2, 5, 6, and 7 (0.075 g) were dissolved in MeOH and were separated from solvent by rotary evaporation and vacuum drying. To each vial (Compounds with SA and controls) PBS (10 mL) was added, and SA release was measured from diluted samples using a UV/Vis spectrophotometer at 294 nm at 1, 2, 3, 4, 6 and 24 hours. A Beer-Lambert calibration plot was prepared using solutions of SA (0-0.05 mg/mL SA). Compound 1 + SA: (1 hour) = 1.137, (2 hour) = 0.248, (3 hour) = 0.120, (4 hour) = 0.247, (6 hour) = 0.136, (24 hour) = 0.651. Compound 2 + SA: (1 hour) = 1.249, (2 hour) = 0.316, (3 hour) = 0.084, (4 hour) = 0.207, (6 hour) = 0.305, (24 hour) = 0.373. Compound 5 + SA: (1 hour) = 0.518, (2 hour) = 0.230, (3 hour) = 0.166, (4 hour) = 0.268, (6 hour) = 0.583, (24 hour) = 0.873. Compound 6 + SA: (1 hour) = 0.882, (2 hour) = 0.364, (3 hour) = 0.218, (4 hour) = 0.282, (6 hour) = 0.424, (24 hour) = 0.687. Compound 7 + SA: (1 hour) = 0.689. The chemical composition and functional groups with in the formulation highlights the tunability of the coating matrix for a desired release profile. The amount of SA in media shows the ability of the platform to not only interact but also release the pharmaceutical component.

### EXAMPLE 35: Release of PTX from Compounds 1, 2, and 6 into Tween PBS.

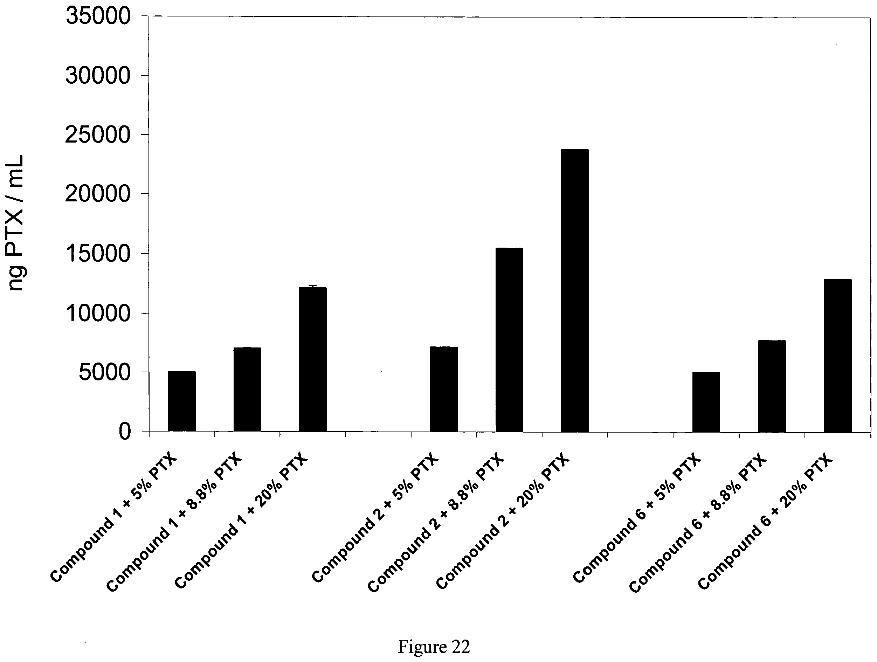

Compounds 1, 2, and 6 were combined with PTX at 5, 8.8 and 20 wt% in DCM, and aliquots of each solution (0.1 mL) were transferred to 4 mL glass vials in duplicate. The solvent was flashed off, and the vials were dried under vacuum at ambient temperatures. Tween PBS (1 mL) was added to each vial, and the vials were incubated at 37°C. After 1 hour, the buffer was withdrawn and PTX content was analyzed by HPLC (Figure 22).

### EXAMPLE 36: Release of PTX from Compounds 1 and 6 into Tween PBS.

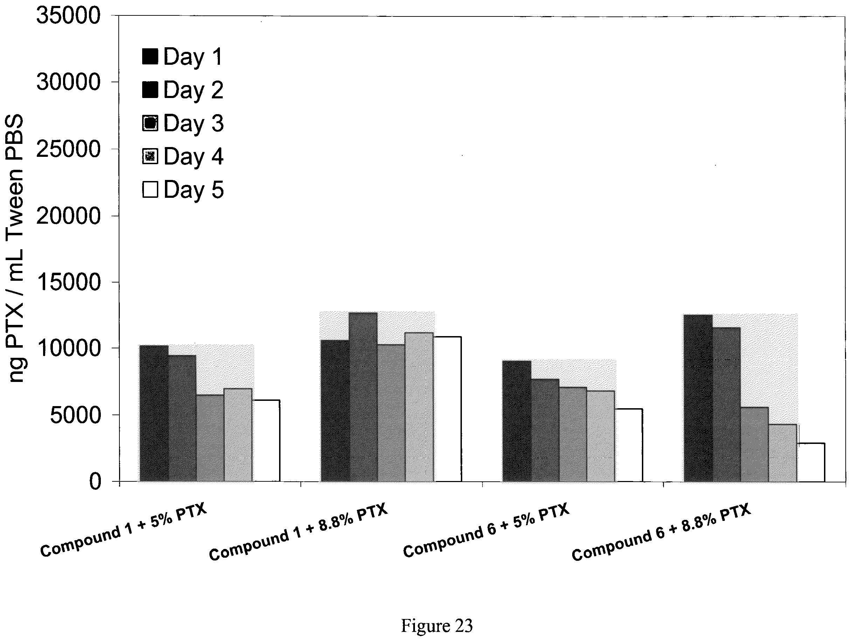

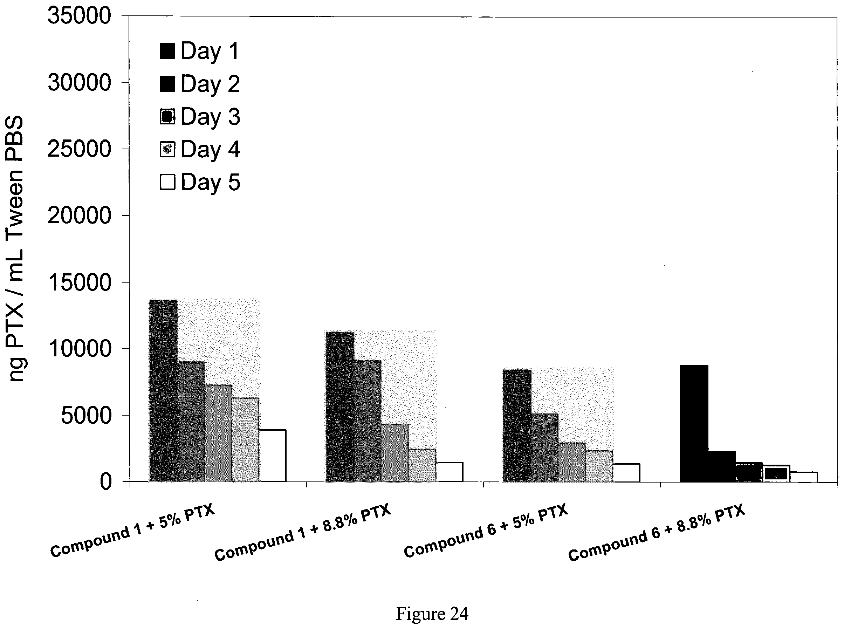

Compounds 1 and 6 were combined with PTX at 5 and 8.8 wt% in DCM, and aliquots of each solution (0.1 mL) were transferred to 4 mL glass vials in duplicate. Tween PBS (1 mL) was added to each vial, and the vials were incubated at 20 and 37°C. At selected time-points (1, 2, 3,4, and 5 days) the buffer was withdrawn for PTX analysis by HPLC (Figures 23 and 24) and replenished with Tween PBS (1 mL).

### EXAMPLE 37: Release of PTX from Compounds 1 and SIBS into Tween PBS.

Compound 1 and SIBS were weighed into 4 mL glass vials as described in Table 1, and were dissolved in THF:toluene. PTX (0.04 g) was dissolved in THF:toluene, and PTX solution was added the Compound 1 and SIBS solutions (0.001 g PTX per vial) and mixed overnight. The solvent was rapidly removed from each vial under vacuum and dried overnight. Tween PBS (1 mL) was added to each vial, and the release of PTX was measured by HPLC after 24 hours (Table 1).

**Table 1: Preparation of PTX-containing mixtures of Compound 1 and SIBS, and release of PTX into Tween PBS.**

| Compound | Mass PTX (g) | Wt% PTX | Release of PTX (ng/mL) after 24 hours |
|---|---|---|---|
| Compound 1 | 0.001 | 0.5 | 5029 |
| Compound 1 | | 1 | 10409 |
| Compound 1 | | 8.8 | 68694 |
| SIBS | | 8.8 | 3356 |

The data shows the enhanced differences in efficiency between conventional base polymer drug release and self eliminating drug release.

### EXAMPLE 38: Preparation of Compound 1 + 8.8 wt% PTX, coating on a stent and release profile of PTX.

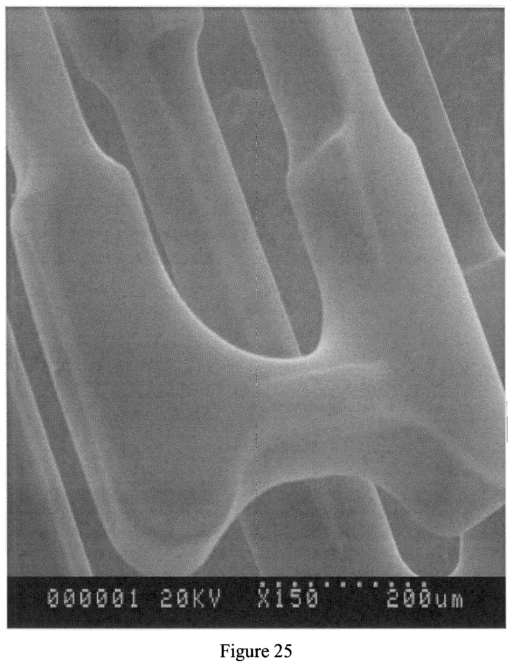

Compound 1 (0.33 g) was dissolved in THF:toluene, mixed with a PTX solution (0.032 g/mL), stirred for 24 hours at room temperature, and used immediately. The solution was sprayed onto stents using an EFD spray system with settings specific to Compound 1 + 8.8 wt% PTX, and the stents were dried in a 50°C flow oven for 20-24 hours. SEM analysis (Figure 25) indicated a thin coating with minimum webbing between struts. Tween PBS or MilliQ water (1 mL) was added to each stent, and incubated at 37°C, with Tween PBS and water changed daily. PTX release was measured using RP-HPLC with benzonitrile as the internal standard: Tween PBS (ng/mL): (day 1) = 9731, (day 2) = 4330, (day 3) = 2523, and water (ng/mL): (day 1) = 2810, (day 2) = 1489, (day 3) = 1146.

### EXAMPLE 39: Preparation of Compound 1, 6, 7, 1+7, 8, 9, and 12 + 1 wt% PTX, coating on a stent and release profile of PTX into Tween PBS and water.

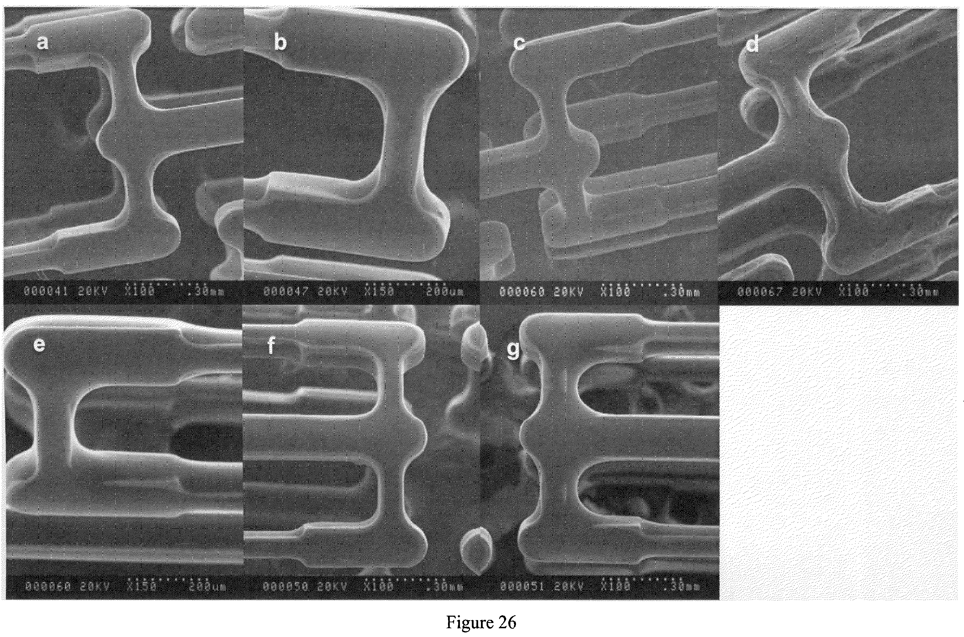

Compounds 1, 6, 7, 1+7, 8, 9, and 12 (0.4 g) were dissolved in THF, mixed with a PTX solution (0.032 g/mL), stirred for 24 hours at room temperature, and used immediately. The solutions were sprayed onto stents using an EFD spray system with settings specific to each Compound + 1 wt% PTX. The stents were dried at room temperature in a fume hood for 4 days. SEM analyses (Figure 26 a-g) indicated thin coatings with minimum webbing between struts for all coatings. PTX release was investigated at 37°C in Tween PBS and in MilliQ water (Tween PBS only for Compound 1+7). Tween PBS or water (1 mL) was added to each stent and changed daily. PTX release was measured using RP-HPLC with benzonitrile as the internal standard: Compound 1 (Tween PBS (ng/mL): (day 1) = 889, (day 2) = 743, (day 3) = 354, and water (ng/mL): (day 1) = 669, (day 2) = 488, (day 3) = 367. Compound 6 (Tween PBS (ng/mL): (day 1) = 1106, (day 2) = 816, (day 3) = 536, (day 4) = 279, (day 5) = 168, and water (ng/mL): (day 1) = 641, (day 2) = 596, (day 3) = 441, (day 4) = 345, (day 5) = 320. Compound 7 (Tween PBS (ng/mL): (day 1) = 1161, (day 2) = 888, (day 3) = 932, (day 4) = 600, (day 5) = 397, (day 6) = 453, (day 7) = 399, (day 8) = 331, (day 9) = 272, and water (ng/mL): (day 1) = 588, (day 2) = 500, (day 3) = 434, (day 4) = 397, (day 5) = 332, (day 6) = 299, (day 7) = 292, (day 8) = 238, (day 9) = 201. Compound 1+7 (Tween PBS (ng/mL): (day 1) = 808, (day 2) = 735, (day 3) = 701, (day 4) = 546. Compound 8 (Tween PBS (ng/mL): (day 1) = 1338, (day 2) = 1040, (day 3) = 878, (day 4) = 571, (day 5) = 409, and water (ng/mL): (day 1) = 928, (day 2) = 593, (day 3) = 681, (day 4) = 681, (day 5) = 646. Compound 9 (Tween PBS (ng/mL): (day 1) = 804, (day 2) = 628, (day 3) = 421, and water (ng/mL): (day 1) = 498, (day 2) = 334, (day 3) = 250. Compound 12 (Tween PBS (ng/mL): (day 1) = 1086, and water (ng/mL): (day 1) = 1717. The chemical composition and functional groups with in the formulation highlights the tunability of the coating matrix for a desired release profile. The amount of PTX in media shows the ability of the platform to not only interact but also release the pharmaceutical component.

### EXAMPLE 40: Release of PTX from Compound 1 + 8.8 wt% PTX on stainless steel coupon after contact with cardiac muscle.

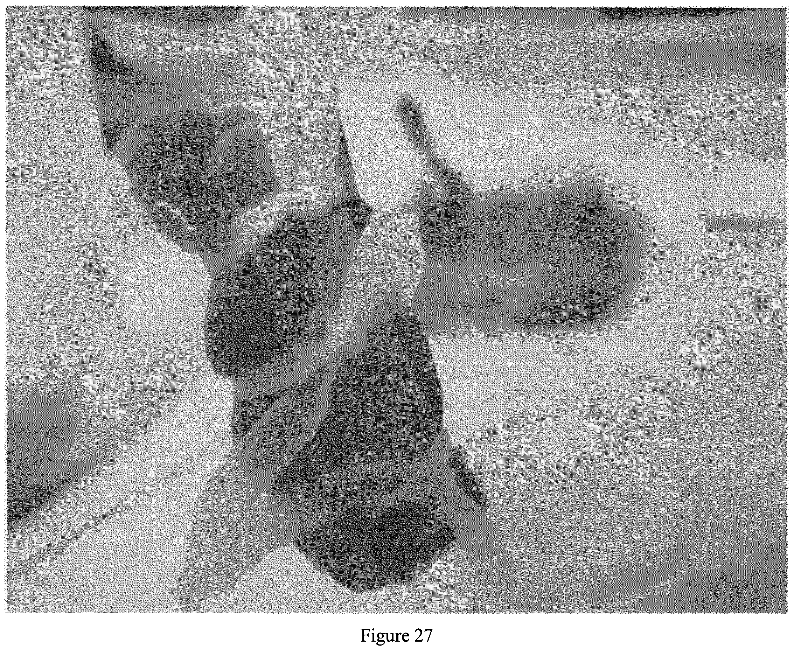

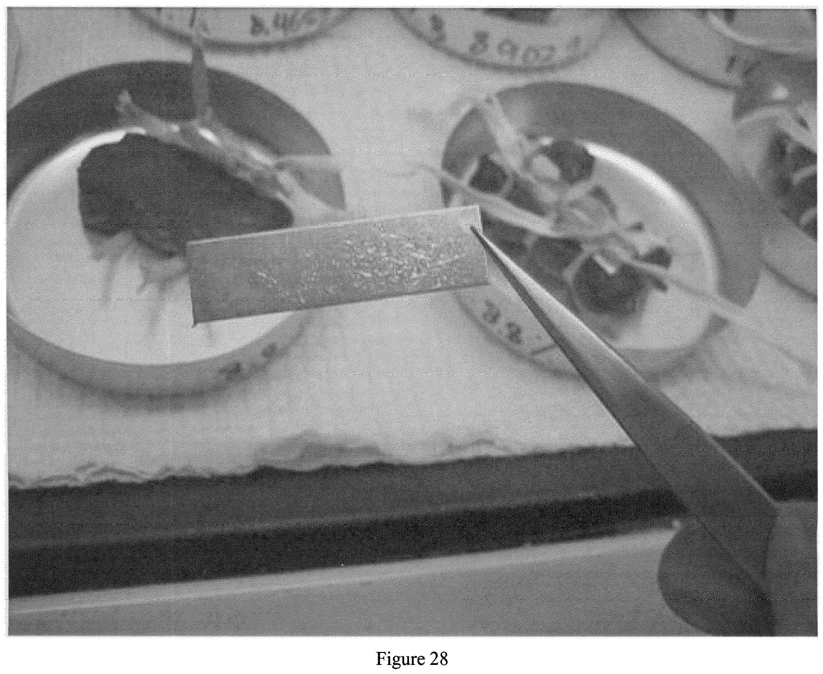

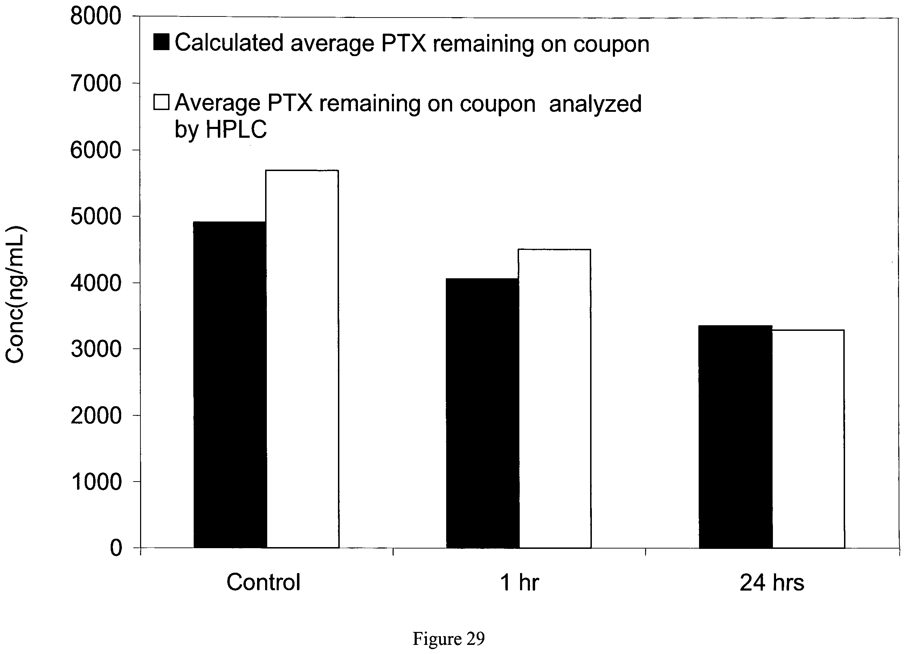

Compound 1 (0.40 g) and PTX (0.0386 g) were dissolved in toluene:THF and stirred overnight. Stainless steel coupons (1.5 g) with dimensions of approximately 3.5 cm x 1 cm were immersed in acetone, sonicated for 45 minutes, and were dried in a 50°C flow oven overnight. One side of each coupon was coated with the solution, and the average mass of Compound 1 + PTX on individual coupons was measured (0.005-0.008 g). The coated sides of the stainless steel coupons were placed in contact with cardiac muscle (7.0-13.0 g), and were secured with umbilical tape (Figure 27). At selected time-points (1 and 24 hours), triplicate test articles and duplicate control articles were removed, rinsed with water, and dried at 50°C overnight in a flow oven (Figure 28). The coating was then stripped off with THF (15 mL) for 3 days, and an aliquot (1 mL) of the stripping solution was submitted for HPLC analysis (acetonitrile: water mobile phase) to determine the average PTX remaining on the coupons (Figure 29).

### EXAMPLE 41: Release of PTX from Compound 1 + 1 wt% PTX on stainless steel coupon after contact with cardiac muscle.

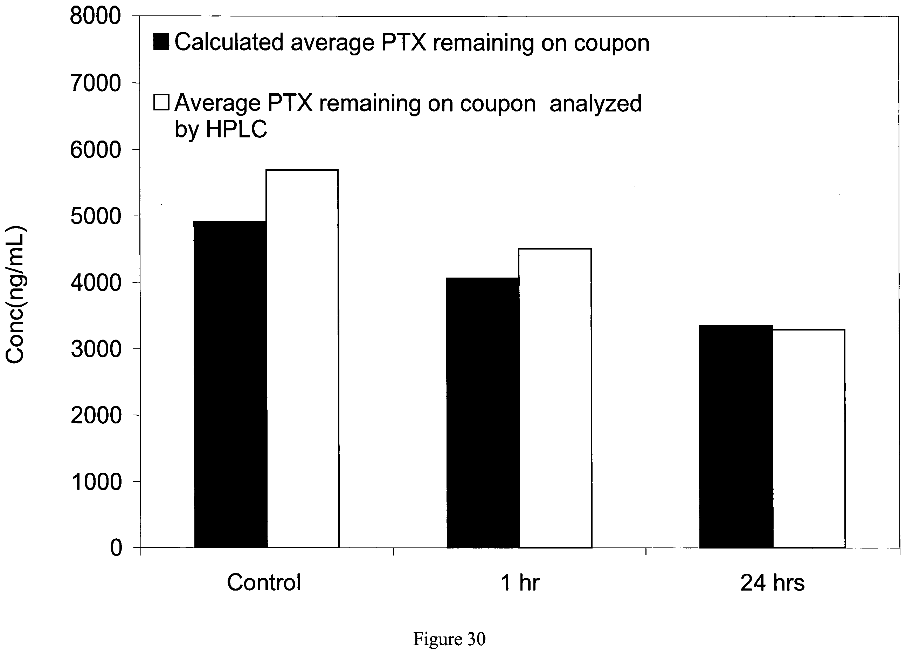

Compound 1 (0.405 g) and PTX (0.004 g) were dissolved in toluene:THF and stirred overnight. Stainless steel coupons (1.5 g) with dimensions of approximately 3.5 cm x 1 cm were immersed in acetone, sonicated for 45 minutes, and dried in 50°C flow oven overnight. One side of each coupon was coated with the solution, and the average mass of Compound 1 + PTX on individual coupons was measured (0.005-0.008 g). The coated sides of the stainless steel coupons were placed in contact with cardiac muscle (7.0-13.0 g), and were secured with umbilical tape. At selected time-points (1 and 24 hours), triplicate test articles and duplicate control articles were removed, rinsed with water, and dried in 50°C flow oven overnight. The coating was then stripped off with THF (15 mL) for 3 days and an aliquot (1 mL) of stripping solution was submitted for HPLC analysis (acetonitrile: water mobile phase) to determine the average PTX remaining on the coupons (Figure 30).

### EXAMPLE 42: Release of PTX from Compound 1 + 8.8 wt% PTX on stainless steel coupon in porcine blood.

Stainless steel coupons prepared as in Example 40 were incubated in porcine blood (4 mL). After 1 hour, triplicate test articles and duplicate control articles were rinsed with water, dried at 50°C overnight, and weighed. The coatings were then stripped off with THF (15 mL) for 3 days and an aliquot
(1 mL) of stripping solution was submitted for HPLC analysis (acetonitrile: water mobile phase) to determine the average PTX remaining on the coupons. Control (ng/mL): (by weight) = 32888, (HPLC) = 38405. 1 hour sample (ng/mL): (by weight) = 34667, (HPLC) = 40962.

### EXAMPLE 43: Residency time of Compounds 1, 2, 3, 6, 7, 1+7, 8, 9, 10, 11, 12, 14, 15 and 16 under sink condition in PBS.

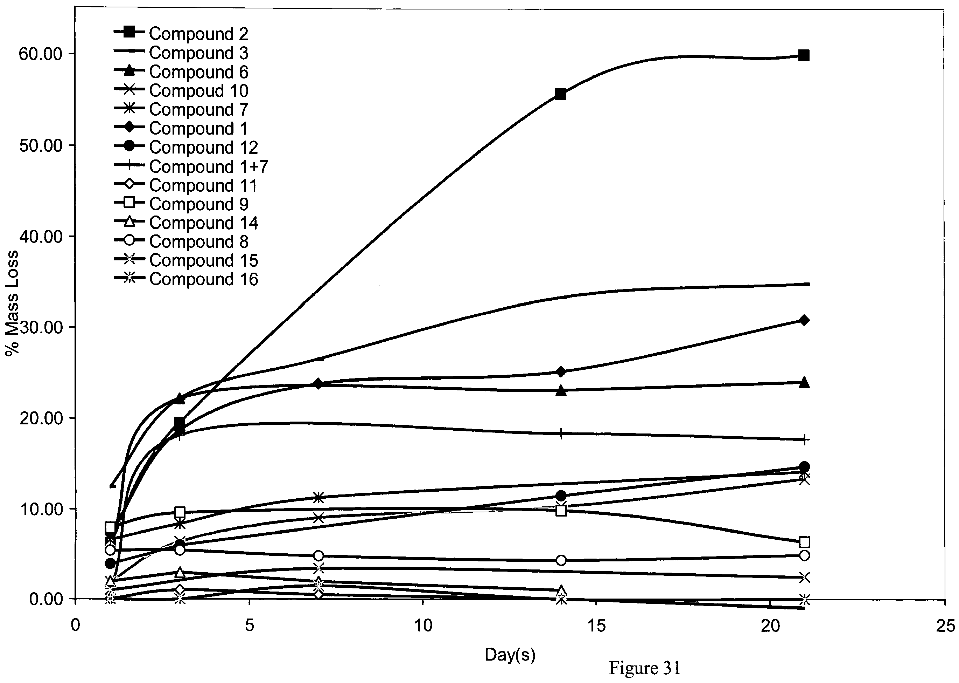

All vials were pre-dried in the oven overnight and tared. Compounds 1, 2, 3, 6, 7, 1+7, 8, 9, 10, 11, 12, 14, 15 and 16 (0.2 g) were weighed into the vials, PBS was added (4 mL), and each vial was then incubated at 37°C. At selected time-points (1, 3, 7, 14 and 21 days) vials were isolated and rinsed with copious amounts of water to remove residual salt, dried in a 50°C flow oven overnight, and weighed. The residency time was calculated by measuring the percentage of mass loss at each time point (Figure 31).

### EXAMPLE 44: Residency of Compounds 1, 2, 1+7, 10 and 12 on stainless steel coupons, under sink condition in PBS.

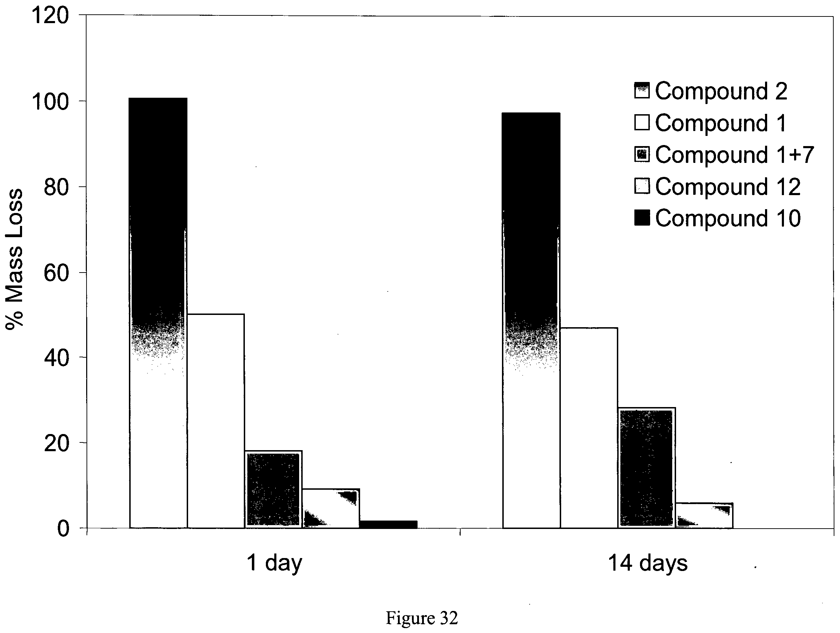

Compound 1 (0.20 g), Compound 10 (0.22 g), and Compound 12 (0.20 g) were dissolved in toluene. Compound 1+7 (0.20 and 0.30 g) was dissolved in THF, and Compound 2 (0.20 g) was dissolved in THF: toluene. Stainless steel coupons (1.5 g) with dimensions of approximately 3.5 cm x 1 cm were immersed in acetone, sonicated for 45 minutes, and dried in a 50°C flow oven overnight. Both sides of the coupons were coated with each Compound solution and the average weight of Compound was measured (0.020-0.040 g). Every coupon was placed in a vial and incubated with PBS (15 mL) at 37°C. At selected time-points (1 and 14 days), the coupons were isolated and rinsed with copious amounts of water and dried in a 50°C flow oven. The residency time was calculated by measuring the percentage of mass loss at each time point (Figure 32).

### EXAMPLE 45: Residency time of Compound 1, 2, 6, 1+7, 10 and 12 under sink condition in porcine blood.

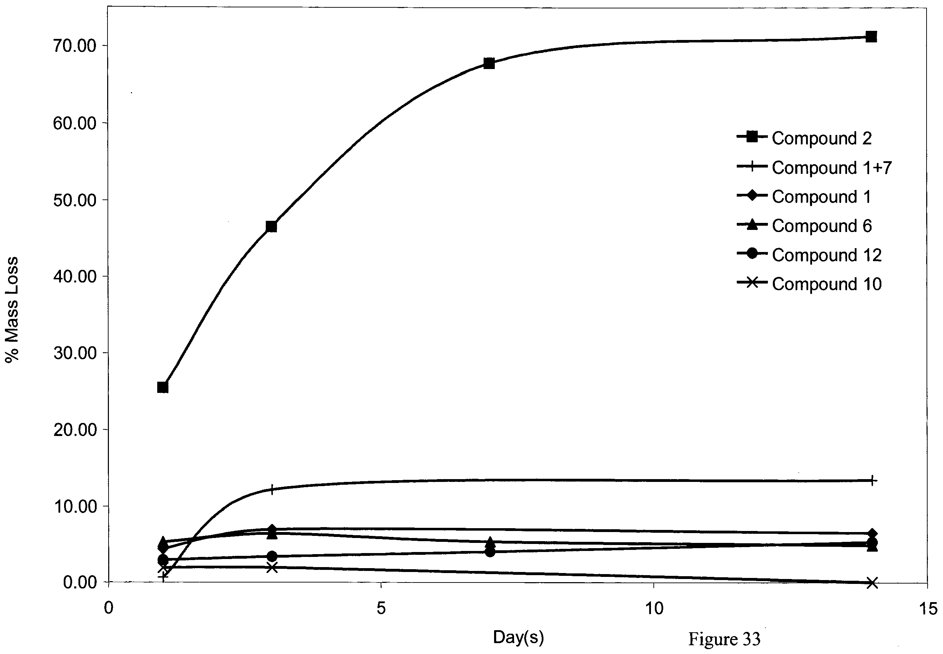

All vials were pre-dried in the oven overnight and tared. Compounds 1, 2, 6, 1+7, 10 and 12 (0.2 g) were weighed into the vials, porcine blood (4 mL) was added to each vial, and incubated at 4°C. At selected time-points (1, 3, 7 and 14 days), vials were isolated and rinsed with copious amounts of water and dried in a 50°C flow oven overnight. The residency time was calculated by measuring the percentage of mass loss after incubation at each time point (Figure 33).

### EXAMPLE 46: Residency time of Compounds 1,2, 10, and 12 on stainless steel coupons under sink condition in porcine blood.

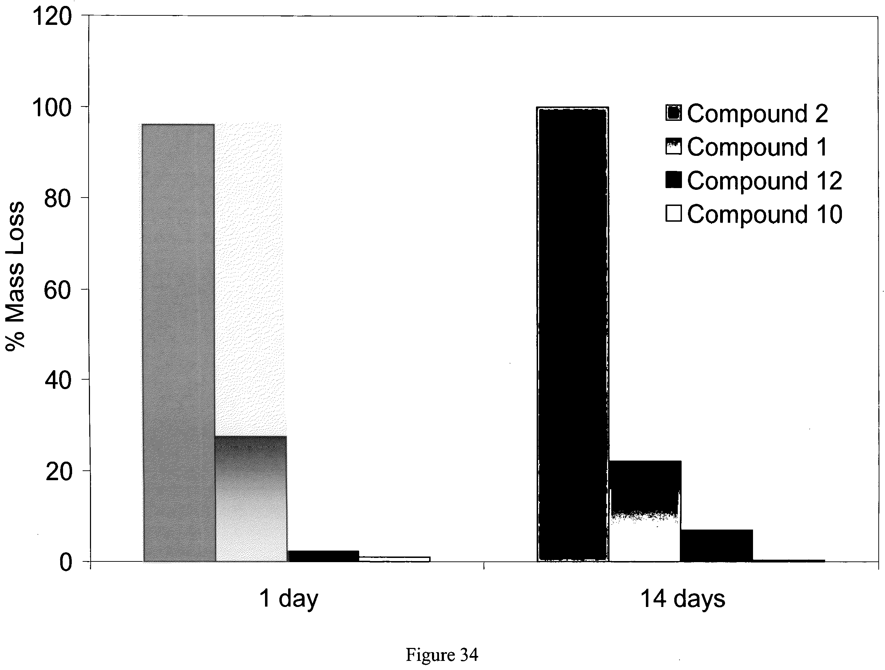

Compound 1 (0.20 g), Compound 10 (0.22 g), and Compound 12 (0.20 g) were dissolved in toluene. Compound 2 (0.20 g) was dissolved in THF: toluene. Stainless steel coupons (1.5 g) with dimensions of approximately 3.5 cm x 1 cm were immersed in acetone, sonicated for 45 minutes, and dried in a 50°C flow oven overnight. Both sides of the coupons were coated with each Compound solution, the average weight of Compound was measured (0.020-0.040 g), and incubated with porcine blood (15 mL) at 4°C. At selected time-points (1 and 14 days) coupons were isolated and rinsed with copious amounts of water and dried in a 50°C flow oven. The residency time was measured by the percentage of mass loss at the end of each time point (Figure 34).

### EXAMPLE 47: Residency time of Compounds 1, 2, 6, 7, 1+7, 10, and 12 under sink condition in artificial urine.

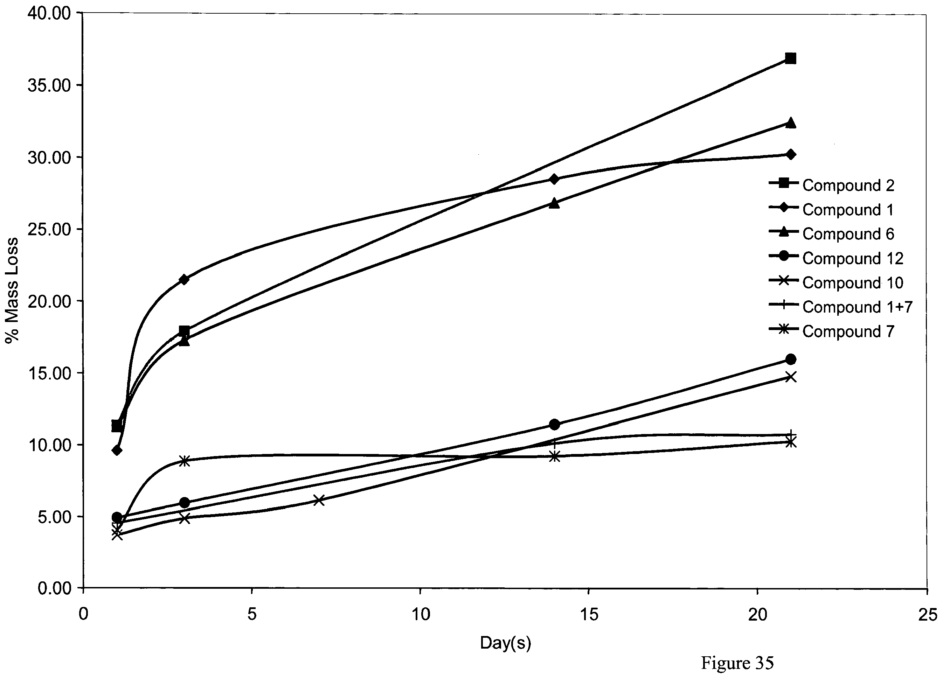

All vials were pre-dried in the oven overnight and tared. Compounds 1, 2, 6, 7, 1+7, 10, and 12 (0.2 g) were weighed in the vials and incubated in artificial urine (4 mL) at 37°C. At selected time-points (1, 3, 7, 14, and 21 days), vials were isolated, rinsed with copious amounts of water, dried in a 50°C flow oven overnight, and weighed. The residency time was calculated by measuring the percentage of mass loss after incubation (Figure 35).

### EXAMPLE 48: Residency time of Compound 1, 2, 4, 6 under flow condition in PBS.

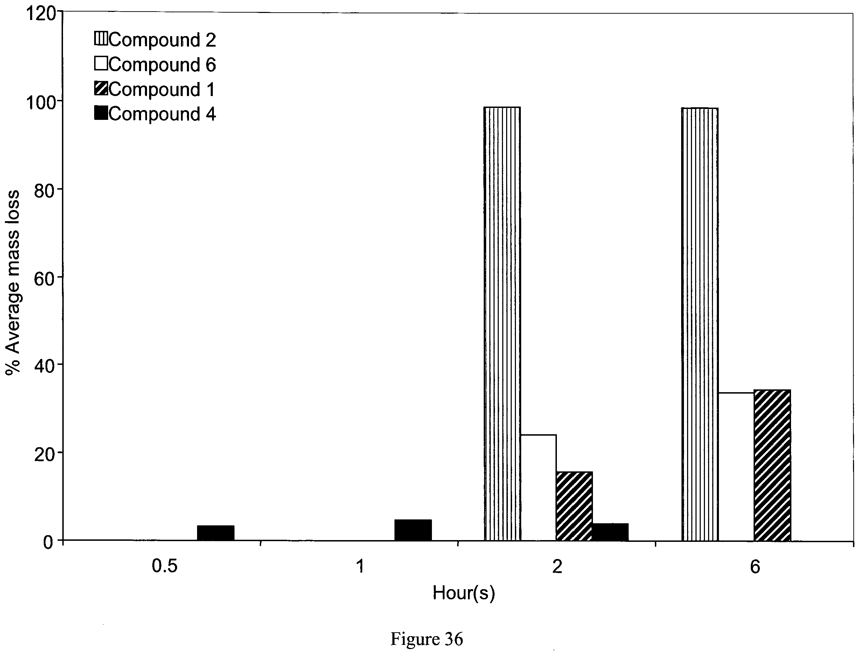

Compounds 1, 2, 4 and 6 (1.05 g) were dissolved in toluene. Stainless steel coupons (1.5 g) with dimensions of approximately 3.5 cm x 1 cm were immersed in acetone, sonicated for 45 minutes, and dried in a 50°C flow oven overnight. Both sides of the coupons were coated with the Compound solutions and the average weight of Compound was measured (0.008-0.012 g). Silicone tubing and PVC tubing were cut 5 cm and 3 cm in length, respectively. Each of the coated coupons were inserted into a silicone tubing segment and labeled. All silicone tubing segments were then connected together by PVC tubing. Clean silicone tubing (100 cm) was wrapped around the head of a peristaltic pump. The outlet was connected to the silicone tubing segments containing coated coupons. PBS was pumped aggressively through the loop system from a PBS reservoir (1000 mL). At selected time-points (2 and 6 hours for Compounds 1, 2, and 6, and 0.5, 1, and 2 hours for Compound 4) triplicate coupons were isolated and rinsed with water several times, dried, and weighed. The residency times were calculated by measuring the percentage of mass loss after each time-point (Figure 36).

### EXAMPLE 49: Residency time of Compound 1, 2 and 6 under flow condition in porcine blood.

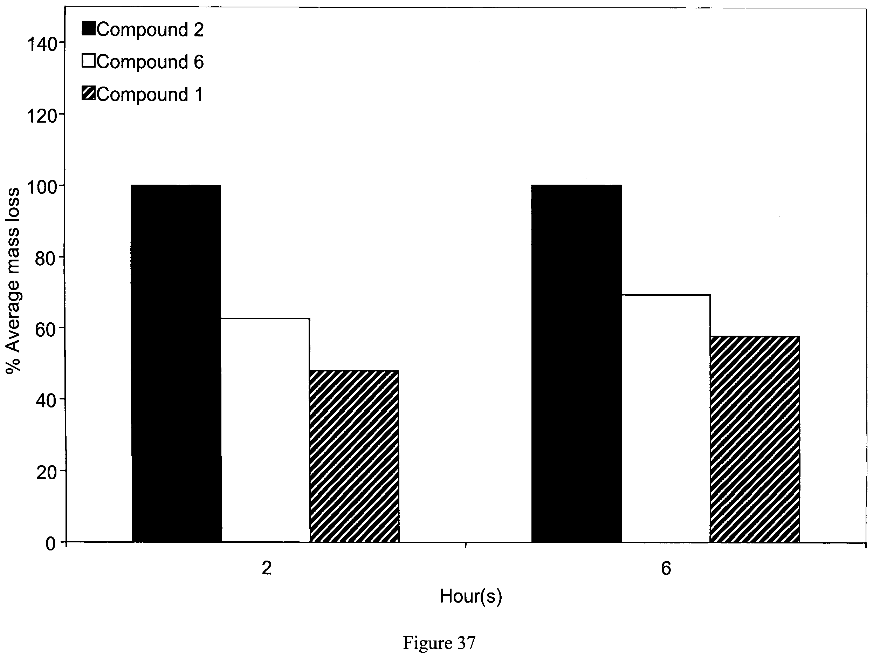

Compounds 1, 2, and 6 (0.50 g) were dissolved in toluene. Stainless steel coupons (1.5 g) with dimensions of approximately 3.5 cm x 1 cm were immersed in acetone, sonicated for 45 minutes, and dried in a 50°C flow oven overnight. Both sides of the coupons were coated with Compound solutions and the average mass of Compound was measured (0.008 to 0.012 g). Silicone tubing and PVC tubing were cut 5 cm and 3 cm in length, respectively. Each of the coated coupons were inserted into a silicone tubing segment and labeled. All silicone tubing segments were then connected together by PVC tubing. Clean silicone tubing (100 cm) was wrapped around the head of a peristaltic pump. The outlet was connected to the silicone tubing segments containing coated coupons. Porcine blood was pumped aggressively through the loop system from a porcine blood reservoir (1000 mL). At selected time-points (2 and 6 hours) triplicate coated coupons and duplicate control coupons were isolated, rinsed with water several times, dried, and weighed. The residency time was calculated by measuring the percentage of mass loss after incubation at each time point (Figure 37).

### EXAMPLE 50: Residency time of Compound 1, 2 and 6 under flow condition in artificial urine.

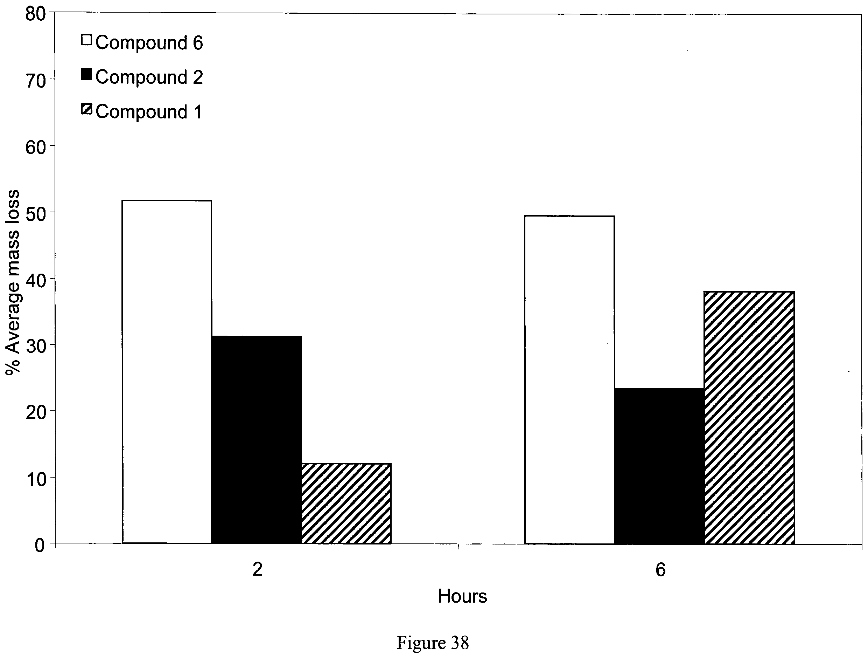

Compounds 1, 2 and 6 (0.50 g) were dissolved in toluene. Stainless steel coupons (1.5 g) with dimensions of approximately 3.5 cm x 1 cm were immersed in acetone, sonicated for 45 minutes, and dried in 50°C flow oven overnight. Both sides of the coupons were coated with Compound solutions and the average mass of Compound was measured (0.008 to 0.012 g). Silicone tubing and PVC tubing were cut 5 cm and 3 cm in length, respectively. Each of the coated coupons were inserted into a silicone tubing segment and labeled. All silicone tubing segments were then connected together by PVC tubing. Clean silicone tubing (100 cm) was wrapped around the head of a peristaltic pump. The outlet was connected to the silicone tubing segments containing coated coupons. Artificial urine was pumped aggressively through the loop system from an artificial urine reservoir (1000 mL). At selected time-points (2 and 6 hours) triplicate coated coupons and duplicate control (uncoated) coupons were isolated, rinsed with water several times, dried, and weighed. The residency time was calculated by measuring the percentage of mass loss after incubation at each time point (Figure 38).

### EXAMPLE 51: Residency time of Compounds 1, 2 and 6 in porcine heart.

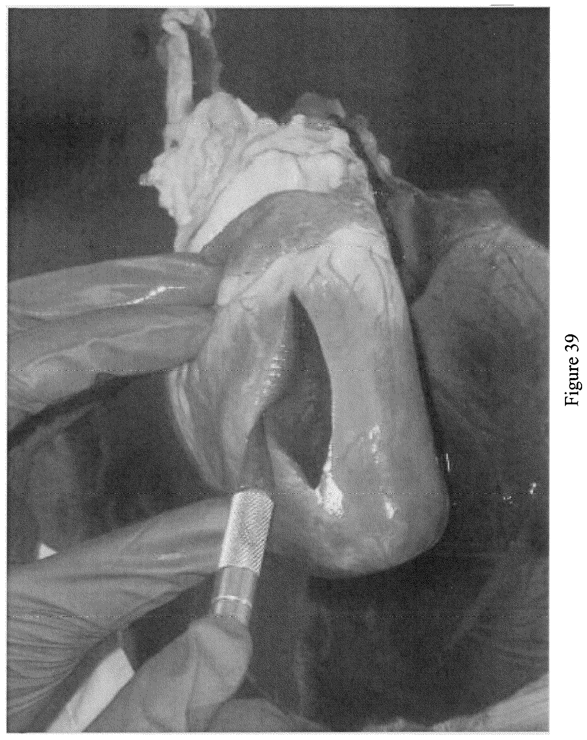

Compounds 1, 2, and 6 (0.5 g) were dissolved in toluene. Stainless steel coupons (1.5 g) with dimensions of approximately 3.5 cm x 1 cm were immersed in acetone, sonicated for 45 minutes, and dried in a 50°C oven overnight. Both sides of the coupons were coated with Compound 1, 2 and 6 solutions and the average mass of Compound was measured (0.008 to 0.012 g). Porcine hearts were thawed and washed with water. A pocket incision 5.5 cm wide was made in the left ventricle of the porcine hearts using a scalpel. The incisions penetrated into the ventricle cavity (Figure 39). Each coated coupon or control article was inserted into the incision, and the hearts were sutured with Ethi-pack 3161 to hold the incision closed. Porcine hearts were placed inside individual plastic containers and placed in a 37°C room. At selected time-points (4 and 24 hours) triplicate coupons and duplicate control coupons (uncoated) were isolated and removed from the porcine hearts, washed with copious amounts of water, dried in a 50°C oven, and weighed. The residency time was calculated by measuring the percentage of mass loss at each time point: Compound 1: (4 hours) = 26.7%, (24 hours) = 63.1 %. Compound 2: (4 hours) = 58.2%, (24 hours) = 63.0%. Compound 6: (4 hours) = 26.6%, (24 hours) = 51.7%.

### EXAMPLE 52: Long term residency study of Compounds 1, 2, 3, 6, 7, 1+7, 8, 9, 10, 11, 12, 14, 15, 16 in PBS sink condition.

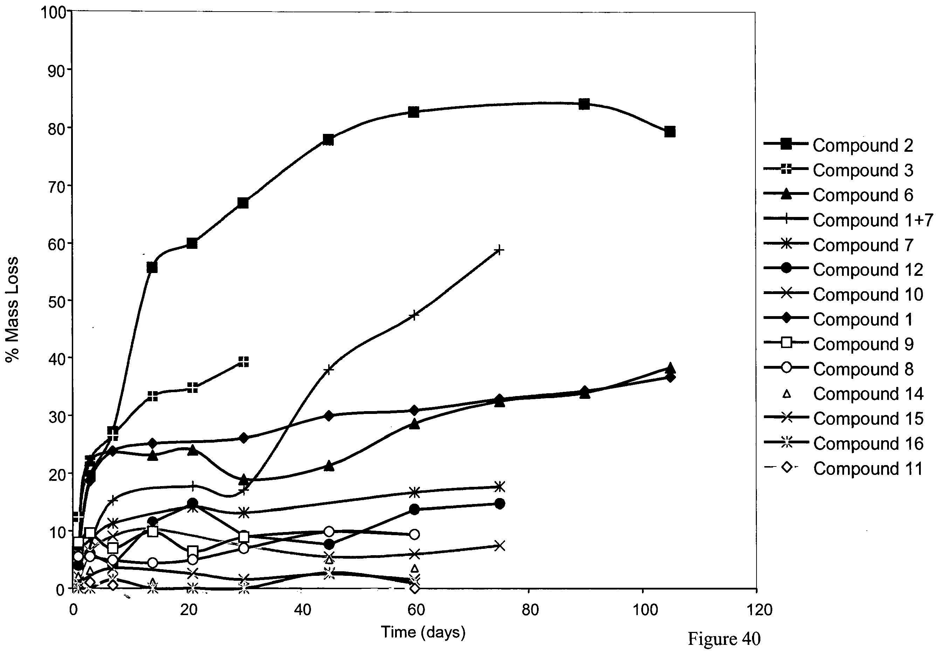

Compounds 1, 2, 3, 6, 7, 1+7, 8, 9, 10, 11, 12, 14, 15, and 16 (0.16-0.20 g) were weighed into pre-dried vials and incubated in PBS (4 mL) at 37°C. At selected time-points (1, 3, 7, 14, 21, 30, 45, 60, 75, 90, 105, 120, 135, 150 days) the PBS was withdrawn, and the Compounds were rinsed with water and dried in a 50°C flow oven overnight. Compounds were weighed the following day and re-incubated with PBS at 37°C. The cycle of PBS removal-water rinsing-drying-re-incubation was repeated until day 150. The residency time was measured by the percentage of mass loss of each Compound at each time point (Figure 40).

### EXAMPLE 53: Determination of the partition coefficient of Compounds 1, 2, 3, 6, 7, 8, 9, 10, 11, 12, SIBS and PTX.

The partitioning of Compounds synthesized in Examples 1-13 was measured in a 1-octanol/PBS system. The partitioning correlates with the lipophilicity of the Compounds, and is used to predict the adsorption and distribution of Compounds under investigation. 1-octanol and PBS were thoroughly mixed to provide PBS-saturated 1-octanol in the top phase and 1-octanol-saturated PBS in the bottom phase. Solutions of Compounds 1, 2, 3, 6, 7, 8, 9, 10, 11, 12, 16, PTX, and SIBS (0.05 mL) were added to each vial of 1-octanol / PBS. The vials were rocked for 4 hours, ensuring good contact between the phases. An aliquot of the organic phase from each sample was then analyzed by GPC (THF mobile phase, RI), which had been calibrated using known concentrations of each compound. The area under the RI peak was used to estimate the concentration of each compound in the organic phase. The mass of each Compound was then used to calculate the weight percentage in organic phase by the following formula: (mass of Compound in 1-octanol phase / total mass) x 100%. The partition coefficient was calculated according to the following formula: log([Compound in 1-octanol]/[Compound in PBS]). Results (wt% in 1-octanol, partition coefficient): Compound 1 (86%, 0.80), Compound 2 (78%, 0.54), Compound 3 (96%, 1.42), Compound 6 (86%, 0.80), Compound 7 (85%, 1.50), Compound 8 (94%, 1.20), Compound 9 (91%, 1.00), Compound 10 (93%, 1.14), Compound 11 (99%, 2.37), Compound 12 (98%, 1.64), SIBS (93%, 1.54), PTX (94%, 1.00).

### EXAMPLE 54: Determination of the solubility of Compounds 1, 2, 6, 7, 8, 9, 12, 14, 15 and 16 in Tween PBS and water.

Compounds 1, 2, 6, 7, 8, 9, 12, 14, 15, and 16 (0.05 g) were incubated in Tween PBS or water for 24 hours at room temperature. Tween PBS solutions and water were withdrawn and filtered through 0.45 µm filter discs and dried in a flow oven at 50°C for 2 days. Dried samples were analyzed by ¹H NMR, and the soft segment assignments were used to quantitatively measure Compound solubility in each environment. ¹H NMR (Reference: 20353.52, Compound 1 (Tween PBS): 251.68, Compound 1 (water): 41.82, Compound 2 (Tween PBS): 818.37, Compound 6 (Tween PBS): 52.32, Compound 6 (water): 24.57, Compound 14 (Tween PBS): 76.84, Compound 15 (Tween PBS): 83.27, Compound 16 (Tween PBS): 90.04). ¹H NMR (Reference: 15434.13, Compound 7 (Tween PBS): 88.32, Compound 7 (water): 17.63). ¹H NMR (Reference: 53909, Compound 8 (water): 23.43). ¹H NMR (Ref: 35697.31, Compound 9 (Tween PBS): 106.42). ¹H NMR (Reference: 19736.11, Compound 12 (Tween PBS): 1001.88, Compound 12 (water): 1804.78).

### EXAMPLE 55: Stability of Compounds 1, 5, 7, and DL-PLGA in different media.

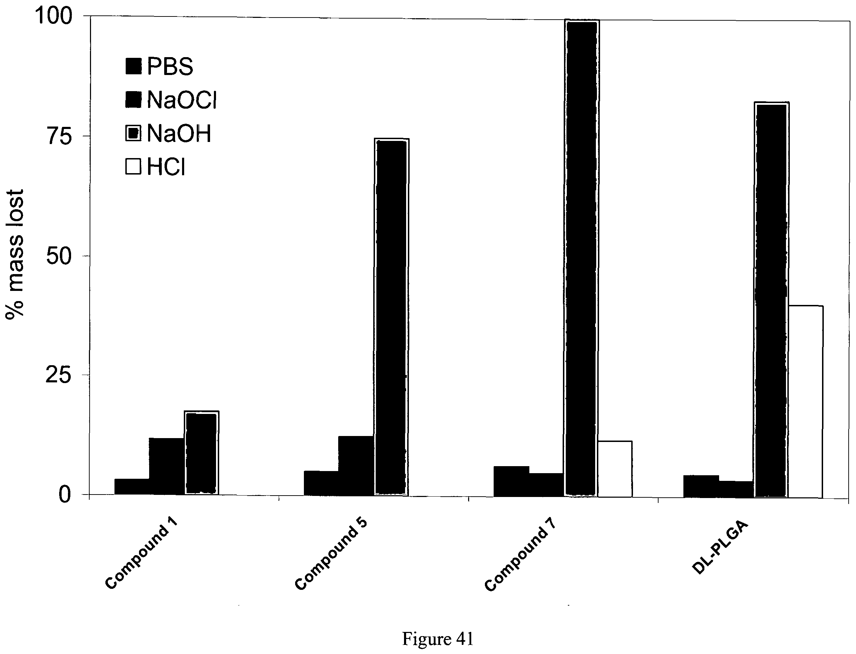

Compounds 1, 5 and 7 (0.06 g x 4 each) were weighed into glass vials and incubated at 37°C for 3 days in PBS, 10 mM NaOCl, 1N NaOH, and 1N HCl (10 mL). DL-PLGA (0.3 g) was dissolved in DCM (1.5 mL), and 0.3 mL of this solution was transferred into glass vials. The solvent was driven off in a 60°C flow oven followed by overnight vacuum drying. The films were then incubated at 37°C for 3 days in PBS, 10 mM NaOCl, 1N NaOH, and 1N HCl (10 mL). The weight change (Figure 41) and GPC profile of each sample was recorded before and after incubation.

### EXAMPLE 56: Sterilization of Compounds 1,1-D, 2, 3, 6, 6-D, 7-D, 8-D, 9-D, 10, 11,12, 14, 15, and 16 by ethylene oxide.

Compounds 1, 2, and 6 (0.115 g) were weighed into a glass vial and melted at 50°C for 30 minutes to form an even layer on the vial bottom. After chilling, the vials were capped with a lint-free tissue, placed in a sterilization pouch, and sterilized by EtO. No changes to the analytical profile were observed. Compounds 1-D, 3, 6-D, 7-D, 8-D, 9-D, 10, 11, 12, 14, 15, and 16 (0.15 g) were weighed into polypropylene conical tubes capped with lint-free tissue, placed in sterilization pouches, and were sterilized by EtO. The sterilized compounds were analyzed, and these results were compared to the pre-sterilization profile. No changes were observed for pre and post sterilization samples. Residual EtO content: ethylene oxide = 2 ppm, ethylene chlorohydrin = 13 ppm, ethylene glycol < 100 ppm. LAL testing: <0.020 EU/mL in the sample extract.

### EXAMPLE 57: Porcine blood interaction with films of Compounds 1, 6, 7, 1+7, 8, 9, 10, 11, 12, 14 and 15.

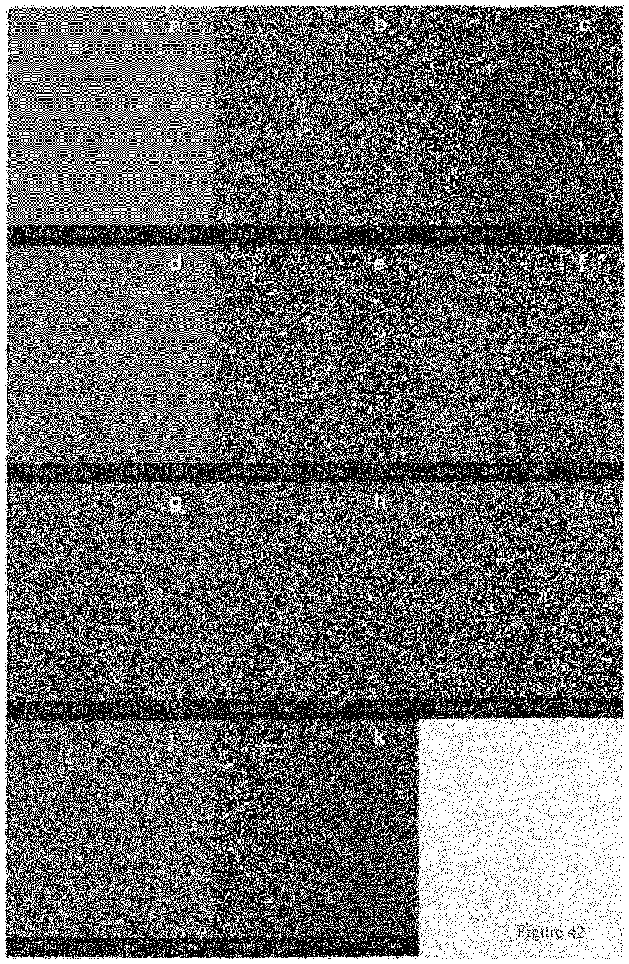

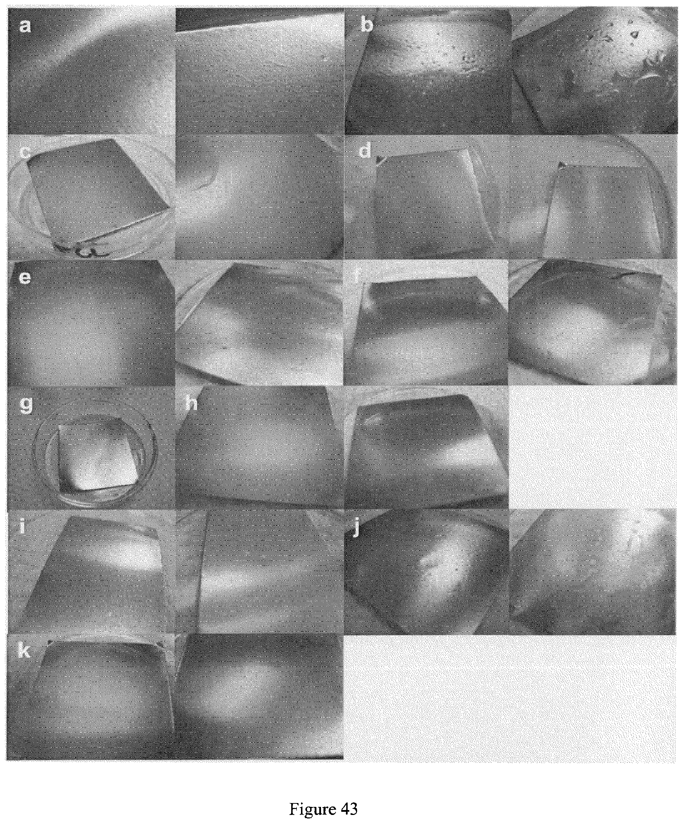

SPINCOATED FILMS: Compound 1 (0.5 g) was dissolved in toluene. A clean 4 x 4 cm stainless steel coupon was placed on the chuck of a Specialty Coating Systems spin-coater, spun at 2000 rpm, and Compound 1 solution (0.15 mL) was applied. The resulting smooth coatings were analyzed by SEM to confirm continuous coverage, and scratched to confirm the presence of coating. XPS analysis (90°) was performed to obtain surface elemental analysis, and to examine for evidence of incomplete coating. Stainless steel: C: 27.84%, Ca: 0.40%, F: 1.18%, Mo: 0.48%, N: 1.18%, Na: 0.46%, O: 52.39% Si: 5.89%, Cr: 3.03%, Fe: 7.15%. Compound 1 coating: C: 51.55%, Ca: 0.36%, F: 31.32%, Mo: 0.02%, N: 3.85%, Na: 0%, Si: 0.06%, Cr: 0%, Fe: 0.01%.
SPRAYED FILMS: Compounds 1 (0.4 g), Compound 6 (0.4 g), Compound 10 (0.2 g), Compound 11 (0.2 g), Compound 12 (0.4 g), Compound 14 (0.2 g), and Compound 15 (0.2 g) were dissolved in toluene. Similarly, Compounds 7, 1+7, 8, and 9 (all 0.4 g) were dissolved in THF. The solutions were stirred for 24 hours at room temperature and were sprayed onto 316 stainless steel coupons ranging in total surface area from 6.25 cm² to 16 cm², using an EFD spray system with settings specific to each Compound. The coupons were dried in a 50°C flow oven for 20-24 hours. SEM images (Figure 42) indicated a smooth, even coating for most compounds except for Compounds 10 and 11. These compounds had a slightly rough but even coating. Contact angle analyses of the coated coupons were performed with water and porcine plasma: stainless steel (water: 59°, plasma: 58°), Compound 1 (water: 107°, plasma: 104°), Compound 7 (water: 88°, plasma: 89°), Compound 1 + 7 (water: 90°, plasma: 85°), Compound 12 (water: 114°, plasma: 119°), Compound 15 (water: 116°, plasma: 116°). No appreciable changes to the coating surfaces were noted when incubated in porcine blood for 15 minutes at room temperature (Figure 43).

### EXAMPLE 58: Evaluation of inflammatory cell response to Compounds 1, 2, 6, 7, 1+7, 8, 9, 15 and 16.

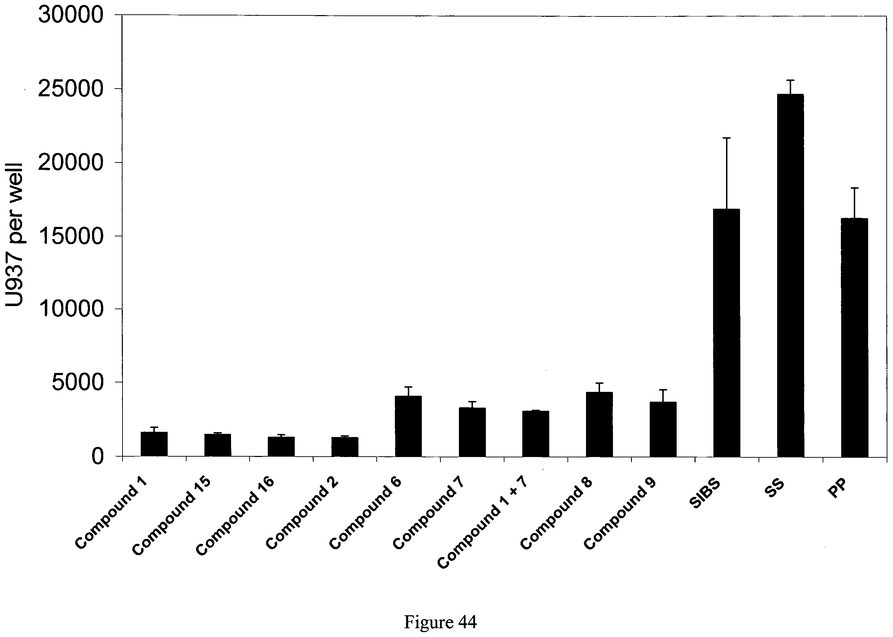

Compounds 1, 2, 6, 7, 1+7, 8, 9, 15, and 16 were dissolved in THF or toluene, and were cast into 96 well polypropylene plates. The solvent was evaporated off at room temperature for 24 hours, the plates were then placed in a 60°C flow oven for 24 hours, and finally dried under vacuum overnight. For comparison purposes, films of SIBS and 316 stainless steel inserts were added to the plates. The plates were sterilized under a UV lamp for 1 hour, after which each sample well was hydrated with PBS. U937 monocyte-like cells (2.5x10⁵ cells) were seeded into each well in the presence of PMA, and the plates were incubated at 37°C in a humid incubator for three days. Non-adherent cells were removed, and adherent U937 macrophages were enumerated using a CyQuant assay (Figure 44). The same experimental procedure was applied to all Compounds described in Examples 1 to 16.

### EXAMPLE 59: Migration of HCAEC through membranes coated with Compound 1, and Compound 1 + 1 and 10 wt% PTX.

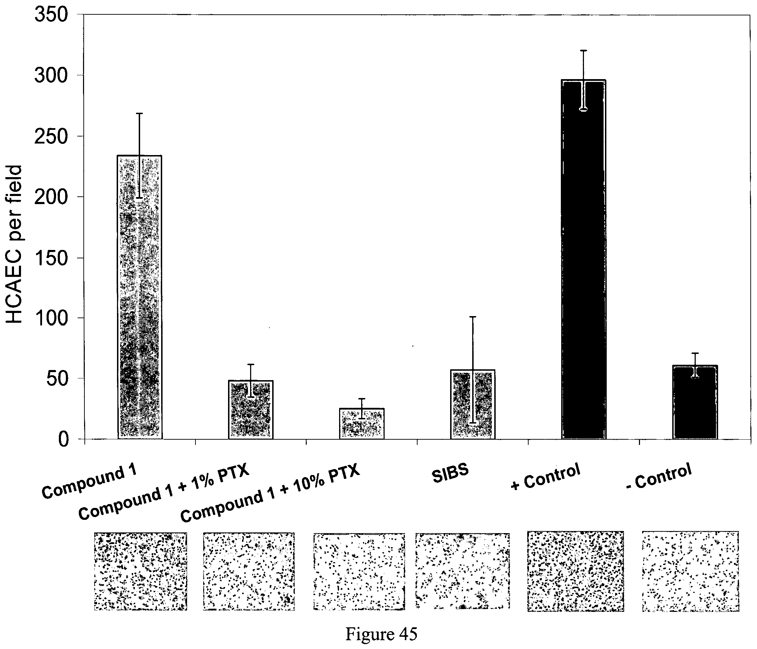

Compound 1 (0.1 g) was dissolved in MeOH (0.5, 1, 2 and 4 mL), and these solutions (0.05 mL) were pipetted onto and wicked through a BD 8 µm PET membrane insert. Compound 1 was also blended with PTX to form 1 and 10 wt% solutions, and these were coated onto membranes. As a control, a solution of SIBS polymer of suitable concentration was coated onto membranes. The resulting coated membranes were examined by SEM and porosity was confirmed by the passage of water through the membranes. Further, Compound 6 (fluorescently labeled form of Compound 1) was coated using the same method, and fluorescence (Ex 320, Em 540 nm) was measured to confirm the presence of coating: (uncoated membrane) = 3.5, (Compound 1) = 0.6, (Compound 6) = 28.2. HCAEC were cultured to third passage using media and supplements supplied by Lonzo, and were starved in serum-free media overnight. Cells were lifted and re-suspended in 0.5% FBS media, and HCAEC were seeded (80 000 per membrane insert). The lower wells were filled with 20% FBS media. The negative control consisted of an uncoated membrane with 0.5% FBS media in the lower well. The positive control consisted of an uncoated membrane with 20% FBS media in the lower well. After four hours of incubation, the wells were lifted out, the inner membranes were scrubbed free of cells, and the lower membrane surface was fixed and stained with DiffQuik. Images of the membranes were collected by microscopy, and cell morphology characteristics and population were recorded (Figure 45). The same experimental procedure was applied to all Compounds described in Examples 1 to 16.

The migration assay as described for the HCAEC was repeated with cell lines from other species on all Compounds described in Examples 1-16.

### EXAMPLE 60: Evaluation of platelet and fibrinogen interaction with films of Compounds 1, 7, 1+7, and 12.

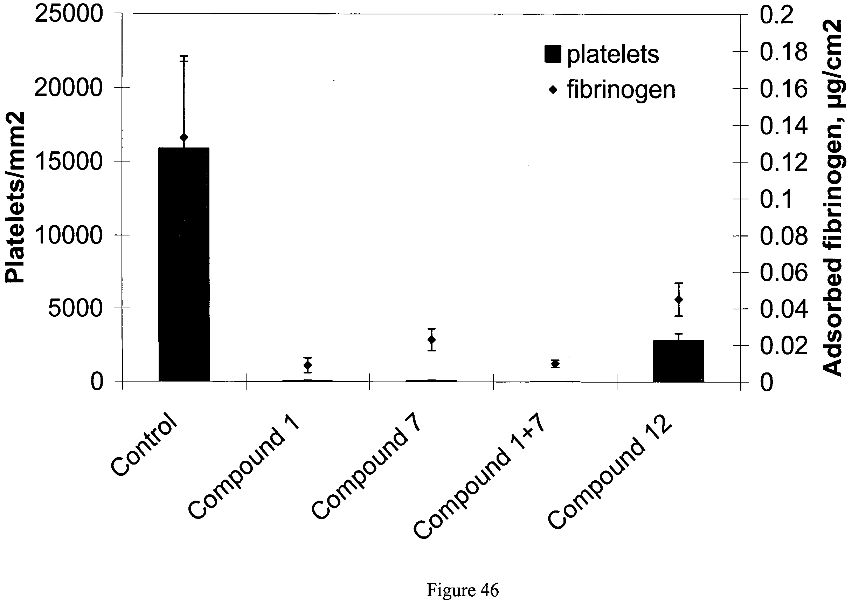

Compounds 1, 7, 1+7, and 12 were dissolved in toluene or THF. All Compound solutions were stirred for 24 hours at room temperature and used for coating. The solutions were sprayed onto 4 cm x 4 cm 316L stainless steel coupons using an EFD spray system with settings specific to each Compound. The coupons were dried in a 50°C flow oven for 20-24 hours. Human whole blood was obtained from healthy drug-free volunteers and collected in centrifuge tubes with either acid citrate dextrose anticoagulant (6 parts ACD to 1 part blood) or low molecular weight heparin to a final concentration of 0.2 U/mL. Platelets from blood collected with ACD were isolated by centrifugation and tagged with 0.5 mCi/mL Na⁵¹Cr. Red blood cells were also isolated from the ACD whole blood and washed. The Na⁵¹Cr tagged platelets and washed red blood cells were combined with platelet poor plasma to give a final platelet concentration of 250,000 platelets/µL and a 40% hematocrit. Finally, ¹²⁵I-fibrinogen was added to the whole blood suspension such that it represented approximately 2% of the total amount of fibrinogen. Platelet adhesion was measured from flowing whole blood in a cone-and-plate device which produces laminar flow and a uniform rate of shear. Coated coupons were placed in the wells of the cone-and-plate device with 1.2 mL of the whole blood suspension containing Na⁵¹Cr platelets and the assay was conducted for 15 minutes. The coupons were then rinsed with fresh buffer and the radioactivity was measured with a γ counter and correlated to the number of adherent platelets (Na⁵¹Cr platelets) and adsorbed fibrinogen (¹²⁵I-fibrinogen) based on the radioactivity of the original whole blood suspension. Compared to the uncoated stainless steel coupon, all coated coupons significantly reduced platelet adhesion and fibrinogen adsorption (Figure 46).

### EXAMPLE 61: MEM elution assay - cytotoxicity assessment of Compounds 1-D, 6-D, 8-D, 9-D, 15, and 16.

Compounds 1-D, 6-D, 8-D, 9-D, 15, and 16 were weighed and incubated in MEM media at a 4 g: 20 mL ratio for 24 hours at 37°C. L-929 mouse fibroblast cells were seeded and incubated at 37°C in 5% CO₂ to obtain sub-confluent monolayers of cells. The growth medium in triplicate cultures was replaced with MEM extract (2 mL). Triplicate cultures were also prepared as positive and negative controls. Cell cultures were examined under microscope after 24 hours to evaluate cellular characteristics and percent lysis. Under the conditions of this test, the MEM extracts showed no evidence of causing cell lysis or toxicity. The same experimental procedure was applied to all Compounds described in Examples 1 to 16.

### EXAMPLE 62: Direct contact assay of Compounds 1-D, 6-D, 8-D, 9-D, 15, and 16.

The viability of HeLa epithelial cells in direct contact with test materials was used to assess the potential cytotoxicity of Compounds 1-D, 6-D, 8-D, 9-D, 15, and 16. Samples of Compounds were solvent cast on agar-supported Supor filters. Subsequently, a monolayer of HeLa cells were cultured directly on the filter, in the presence of MEM culture media. After 24 hours of incubation, the Supor filter was rinsed and stained with succinic dehydrogenase. Viable cells were identified by a positive purple stain and cytotoxicity was determined by examining the stained filter for cell exclusion zones around the cast material, or a low cell density. Each cytotoxicity assay included a positive and negative control. The same experimental method was applied to all Compounds described in Examples 1-16.

### EXAMPLE 63: Partial thromboplastin assay of Compound 1-D.

Compound 1-D was weighed and exposed to citrated human plasma at a ratio of 0.2 g test sample to 1 mL of plasma for 15 minutes at 37°C. At the end of incubation period, a PTT reagent of Rabbit Brain Cephalin (RBC) and an activator reagent (calcium chloride) were added to the plasma samples. The plasma samples were then analyzed on the Cascade M-4 manual coagulation analyzer for the time required to form a clot. By this assay, Compound 1-D was considered to be a non-activator of the intrinsic pathway. The same experimental method was applied to all Compounds described in Examples 1-16.

### EXAMPLE 64: In vivo studies in porcine model.

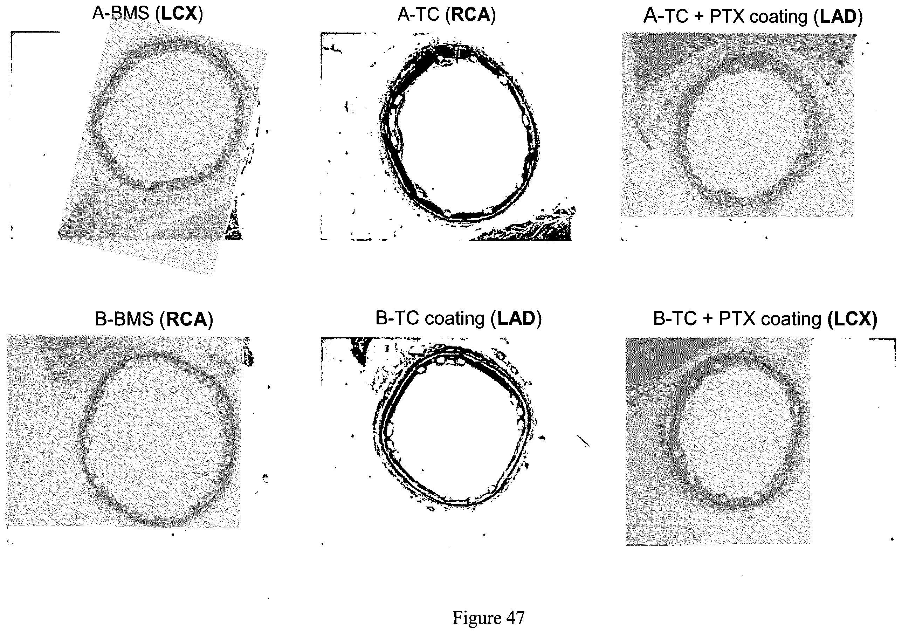

Stents of different types (bare metal, test Compound, and test Compound + 1% PTX) were implanted in porcine coronary arteries (castrated male farm porcines, Sus scrofa domestica; weight 40-50 kg at time of stent implantation). Each animal received ASA (0.081 g) and Clopidogrel (0.075 g) by mouth daily for three days prior to stent implant, and were fasted overnight before the procedure. For surgical procedures, after sedation a marginal ear vein was cannulated for infusion of intravenous fluids and medications. The animal was intubated for administration of anesthetic gases and placed on the catheterization table. Under sterile conditions, a vascular introducer sheath was placed in the right femoral artery by surgical cut down. Continuous hemodynamic monitoring and electrocardiographic monitoring was maintained throughout the procedure. Using the guide catheter as a calibration reference, the diameter of the vessel at reference sites proximal and distal to the intended site of implant, as well as the target site diameter, was measured. Stents were implanted in LAD, RCA and LCX arteries in each animal and were sized ∼15% larger than the target site diameter by balloon catheter expansion. At termination seven days after stent implantation, the animals were euthanized, the heart was excised and examined for any abnormalities, and the vasculature was perfused with saline solution to clear the blood. Hearts were kept immersed in fresh formalin until excision of stents for microscopic analysis (Figure 47). At termination, the liver, spleen and kidneys from test animals were harvested for further analysis. A careful analysis of multiple sections of these organs revealed no remarkable pathological features. The tissue from all three organs showed normal architecture and did not exhibit any sign of inflammation or tissue injury.

### EXAMPLE 65: Compound 3 - Coating quality, stability and integrity on stents.

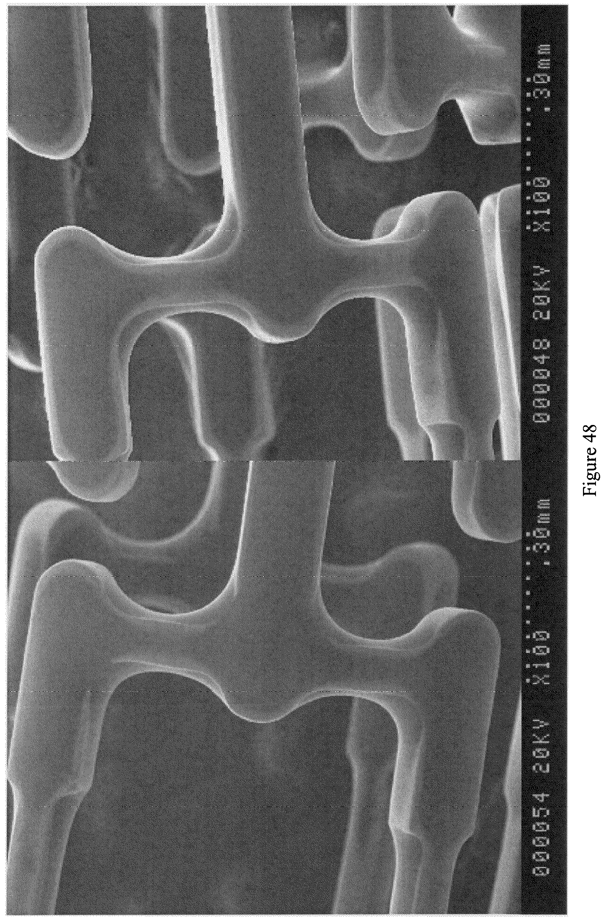

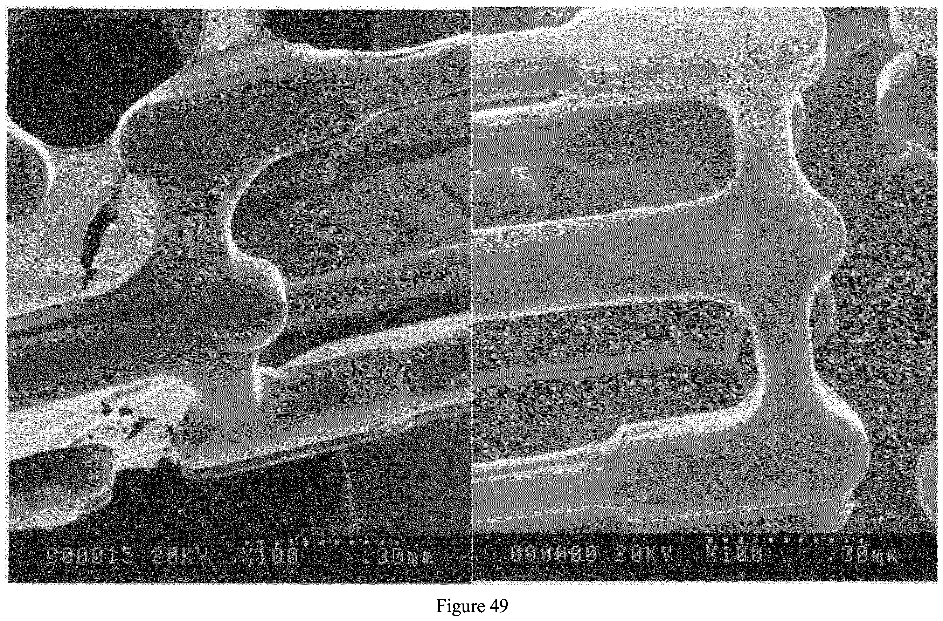

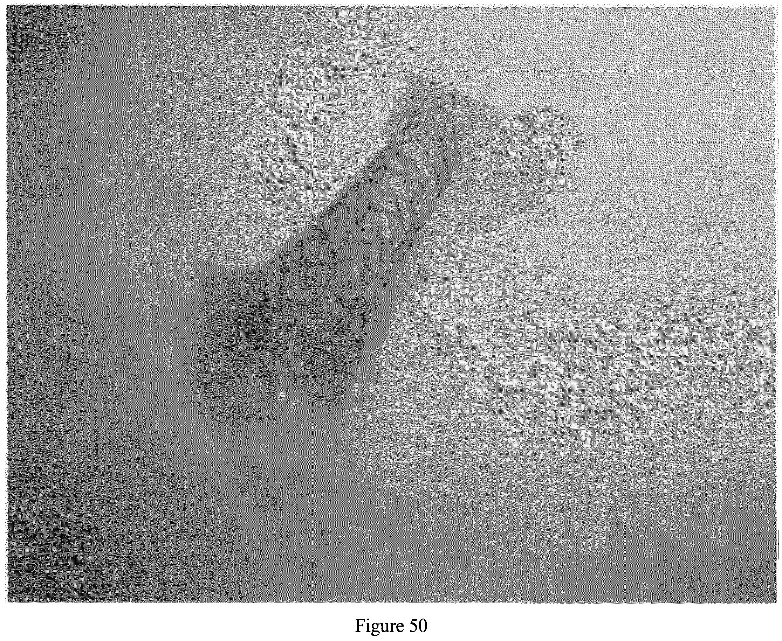

COATING QUALITY: Compound 3 (0.2 g) was dissolved in THF: toluene, was stirred for 24 hours at room temperature, and the solution was sprayed onto stents using an EFD spray system using settings specific to Compound 3. The stents were dried in a 50°C flow oven for 20-24 hours. The stent coating was assessed for uniform strut coverage by SEM. Stents were then either crimped and deployed or kept as cut. After processing, the coatings on both stents and balloons were assessed by microplate analysis (Ex 320 nm, Em 540 nm). Coating quantity was measured directly on both the stent (as cut: 97.88, deployed: 54.39) and balloon (36.69), as well as after stripping with MeOH for 24 hours (stent as cut: 47.03, deployed: 29.79, balloon: 10.55). Transfer of Compound 3 to the balloon during deployment was examined by UV light.
COATING STABILITY: Stents were coated with Compound 3 as above and stored in the dark at room temperature for 42 days. Stent coatings were stripped in MeOH and the coating was quantified using a microplate reader (Ex 320 nm, Em 540 nm) after 1 and 42 days in storage. The microplate readings were similar regardless of storage time ((1 day) = 40.85, (42 days) = 39.80). SEM confirmed that the coating on the stent did not change after 42 days in storage (Figure 48). Similarly, stents were coated with Compound 3 as above and were incubated in porcine blood (1.5 mL) at 37°C on a shaker (60 rpm), with daily changes of the blood. After 7 days stents were examined with SEM (Figure 49-right). Stents were also incubated in porcine blood (1.5 mL) at 4°C for 24 hours with the blood changed after 4, 8 and 24 hours. SEM of these stents revealed a considerable amount of protein deposition, and minimal loss of coating (Figure 49-left). The coating was also evaluated using microplate analysis (Ex: 320 nm, Em: 540 nm): (0 hour) = 77.18, (1 hour) = 71.43, (4 hours) = 67.74, (24 hours) = 37.16, (7 days) = 24.64.
COATING INTEGRITY: Stents were coated with Compound 3 as above and crimped on balloons. A female pig (20 lb) was implanted with a Compound 3 coated stent in each of the following arteries: left anterior descending artery (LAD), left circumflex artery (LCX), and right coronary artery (RCA). Stents were explanted 45-90 minutes after implantation (Figure 50). LAD and LCX explanted stents were stripped in MeOH for coating quantification by microplate analysis (Ex: 320 nm, Em: 540 nm): (LAD) = 29.62, (LCX) = 28.12.

## Claims

1. An implantable medical device having a surface and a matrix coating applied to said surface of said implantable medical device, said matrix coating consisting of components having a molecular weight of less than 20 kDa, said matrix coating comprising
(i) an oligomer having a structure described by formula (II):
F_{T} - [B - (oligo)]ₙ- B - (F_{T})_{g} (II)
wherein
B comprises a urethane;
oligo comprises polypropylene oxide, polyethylene oxide, polycarbonate, or polytetramethyleneoxide;
F_{T} is an oligofluoro group;
g is 1; and
n is an integer from 1 to 10;
and
(ii) a biologically active agent, wherein
(a) said matrix coating is self-eliminating or bioerodible upon implantation into a subject,
(b) said biologically active agent, when on the implantable medical device, resides solely within said matrix coating;
(c) said matrix coating does not have the properties of a base polymer;
(d) said matrix coating is applied to said surface by spray coating, printing, or dip coating said implantable medical device; and
(e) said matrix coating thickness is from 0.05 to 15 microns.

2. The implantable medical device of claim 1, wherein said surface is a material selected from metals, metal alloys, ceramics, base polymers, and glasses, or
wherein said biologically active agent is selected from proteins, peptides, carbohydrates, antibiotics, antiproliferative agents, rapamycin macrolides, analgesics, anesthetics, antiangiogenic agents, antithrombotic agents, vasoactive agents, anticoagulants, immunomodulators, cytotoxic agents, antiviral agents, antibodies, neurotransmitters, psychoactive drugs, oligonucleotides, vitamins, lipids, and prodrugs thereof.

3. The implantable medical device of claim 1, wherein said biologically active agent is complexed to said oligomer.

4. The implantable medical device of any one of claims 1-3, wherein F_{T} has the formula:
CF₃(CF₂)ₚX,
(CF₃)₂CF(CF₂)ₚX,
or
(CF₃)₃C(CF₂)ₚX,
wherein X is CH₂CH₂-, (CH₂CH₂O)ₙ, CH₂CH(OH)CH₂O-,
CH₂CH(CH₂OH)O-, or a bond;
p is an integer between 2 and 20; and
n is an integer between 1 and 10.

5. The implantable medical device of any one of claims 1-4, wherein said device is selected from cardiac-assist devices, catheters, stents, prosthetic implants, artificial sphincters, and drug delivery devices, or
wherein said biologically active agent is uniformly distributed throughout said matrix coating, or wherein said biologically active agent is dissolved in said matrix coating, or wherein said matrix coating has a thickness of 0.1 to 5 microns.

6. A method for making a coated implantable medical device according to any one of claims 1-5 having a surface, said method comprising the step of coating said surface with a matrix coating consisting of components having a molecular weight of less than 20 kDa, said matrix coating comprising (i) an oligomer and (ii) a biologically active agent,
wherein said matrix coating is self-eliminating or bioerodible upon implantation into a subject and wherein said biologically active agent, when on the implantable medical device resides solely within said matrix coating.

7. The method of claim 6, wherein said oligomer when on the implantable medical device resides solely within said matrix coating.

8. The method of claim 6 or 7, wherein said step of coating comprises brushing, printing, spraying, wiping, or dipping said surface with said matrix coating, or
wherein said step of coating comprises dissolving the constituents of said matrix coating in a solvent to form a solution and applying said solution to the surface of said implantable medical device, or
wherein said step of coating comprises mixing the constituents of said matrix with a diluent to form a fluid mixture and applying said fluid mixture to the surface of said implantable medical device, or
wherein said implantable medical device is selected from cardiac-assist devices, catheters, stents, prosthetic implants, artificial sphincters, and drug delivery devices, or
wherein said biologically active agent is uniformly distributed throughout said matrix coating, or
wherein said biologically active agent is dissolved in said matrix coating, or wherein said step of coating comprises applying said matrix coating in a thickness of 0.1 to 5 microns, or
wherein said uncoated implantable medical device is coated to produce a coated implantable medical device, said coated implantable medical device having, upon implantation into an animal, reduced protein deposition, reduced fibrinogen deposition, reduced platelet deposition, or reduced inflammatory cell adhesion in comparison to said uncoated implantable medical device.

9. The implantable medical device of any one of claims 1-5, wherein said implantable medical device is a stent, and wherein said biologically active agent is selected from antiproliferative agents and rapamycin macrolides.

10. The stent of claim 9, wherein said matrix coating further comprises a biologically active agent selected from antiproliferative agents, such as methotrexate, trimetrexate, gemcitabine, vinblastine, vincristine, etoposide, teniposide, topotecan, irinotecan, camptothecin, 9-aminocamptothecin, paclitaxel, docetaxel, daunorubicin, doxorubicin, dactinomycin, idarubicin, bleomycin, tamoxifen; and rapamycin macrolides, such as rapamycin, CCI-779, Everolimus, and ABT-578.

11. A stent of claim 9 or 10 for inhibiting restenosis at a site in a vessel.

12. An implantable medical device of any one of claims 1-5 for delivering a biologically active agent to a subject.

## Patentansprüche

1. Implantierbare medizinische Vorrichtung mit einer Oberfläche und einer Matrixbeschichtung, die auf diese Oberfläche dieser implantierbaren medizinischen Vorrichtung aufgetragen ist, wobei diese Matrixbeschichtung aus Bestandteilen besteht, die ein Molekulargewicht von weniger als 20 kDa haben, wobei diese Matrixbeschichtung umfasst
(i) ein Oligomer mit einer Struktur, die durch Formel (II) beschrieben ist:
F_{T} - [B - (Oligo)]ₙ- B - (F_{T})_{g} (II),
wobei
B ein Urethan umfasst;
Oligo Polypropylenoxid, Polyethylenoxid, Polycarbonat oder Polytetramethylenoxid umfasst;
F_{T} eine Oligofluorgruppe ist;
g 1 ist; und
n eine ganze Zahl von 1 bis 10 ist;
und
(ii) einen biologisch aktiven Wirkstoff, wobei
(a) diese Matrixbeschichtung bei Implantation in ein Subjekt selbsteliminierend oder bioerodierbar ("bioerodible") ist,
(b) dieser biologisch aktive Wirkstoff, wenn er sich auf der implantierbaren medizinischen Vorrichtung befindet, nur innerhalb dieser Matrixbeschichtung verbleibt;
(c) diese Matrixbeschichtung nicht die Eigenschaften eines Basispolymers aufweist;
(d) diese Matrixbeschichtung auf diese Oberfläche durch Sprühbeschichtung, Drucken oder Tauchbeschichtung dieser implantierbaren medizinischen Vorrichtung aufgetragen ist; und
(e) die Dicke dieser Matrixbeschichtung von 0,05 bis 15 Mikron ist.

2. Implantierbare medizinische Vorrichtung von Anspruch 1, wobei diese Oberfläche ein Material ist, ausgewählt aus Metallen, Metalllegierungen, Keramik, Basispolymeren und Gläsern, oder
wobei dieser biologisch aktive Wirkstoff ausgewählt ist aus Proteinen, Peptiden, Kohlenhydraten, Antibiotika, antiproliferativen Wirkstoffen, Rapamycin-Makroliden, Analgetika, Anästhetika, antiangiogenen Wirkstoffen, antithrombotischen Wirkstoffen, vasoaktiven Wirkstoffen, Antikoagulantien, Immunmodulatoren, zytotoxischen Wirkstoffen, antiviralen Wirkstoffen, Antikörpern, Neurotransmittern, psychoaktiven Wirkstoffen, Oligonukleotiden, Vitaminen, Lipiden und Prodrugs davon.

3. Implantierbare medizinische Vorrichtung von Anspruch 1, wobei dieser biologisch aktive Wirkstoff an dieses Oligomer komplexiert ist.

4. Implantierbare medizinische Vorrichtung von einem beliebigen der Ansprüche 1-3, wobei F_{T} die Formel hat:
CF₃(CF₂)ₚX,
(CF₃)₂CF(CF₂)ₚX
oder
(CF₃)₃C(CF₂)ₚX,
wobei X CH₂CH₂-, (CH₂CH₂O)ₙ, CH₂CH(OH)CH₂O-,
CH₂CH(CH₂OH)O- oder eine Bindung ist;
p eine ganze Zahl zwischen 2 und 20 ist; und
n eine ganze Zahl zwischen 1 und 10 ist.

5. Implantierbare medizinische Vorrichtung von einem beliebigen der Ansprüche 1-4, wobei diese Vorrichtung ausgewählt ist aus Herzunterstützungsvorrichtungen, Kathetern, Stents, prothetischen Implantaten, künstlichen Schließmuskeln und Arzneistoffabgabevorrichtungen, oder
wobei dieser biologisch aktive Wirkstoff einheitlich durch diese Matrixbeschichtung hindurch verteilt ist oder wobei dieser biologisch aktive Wirkstoff in dieser Matrixbeschichtung gelöst ist oder wobei diese Matrixbeschichtung eine Dicke von 0,1 bis 5 Mikron hat.

6. Verfahren zum Herstellen einer beschichteten implantierbaren medizinischen Vorrichtung gemäß einem beliebigen der Ansprüche 1-5 mit einer Oberfläche, wobei dieses Verfahren den Schritt des Beschichtens dieser Oberfläche mit einer Matrixbeschichtung umfasst, die aus Komponenten besteht, die ein Molekulargewicht von weniger als 20 kDa haben, wobei diese Matrixbeschichtung (i) ein Oligomer und (ii) einen biologisch aktiven Wirkstoff umfasst,
wobei diese Matrixbeschichtung bei Implantation in ein Subjekt selbsteliminierend oder bioerodierbar ist und wobei dieser biologisch aktive Wirkstoff, wenn er sich auf der implantierbaren medizinischen Vorrichtung befindet, nur innerhalb dieser Matrixbeschichtung verbleibt.

7. Verfahren von Anspruch 6, wobei dieses Oligomer, wenn es sich auf der implantierbaren medizinischen Vorrichtung befindet, nur innerhalb dieser Matrixbeschichtung verbleibt.

8. Verfahren von Anspruch 6 oder 7, wobei dieser Schritt des Beschichtens Bürsten, Drucken, Sprühen, Wischen oder Eintauchen dieser Oberfläche mit dieser Matrixbeschichtung umfasst, oder
wobei dieser Schritt des Beschichtens Auflösen der Bestandteile dieser Matrixbeschichtung in einem Lösungsmittel, um eine Lösung zu bilden, und Auftragen dieser Lösung auf die Oberfläche dieser implantierbaren medizinischen Vorrichtung umfasst, oder
wobei dieser Schritt des Beschichtens Mischen der Bestandteile dieser Matrix mit einem Verdünnungsmittel, um ein flüssiges Gemisch zu bilden, und Auftragen dieses flüssigen Gemisches auf die Oberfläche dieser implantierbaren medizinischen Vorrichtung umfasst, oder
wobei diese implantierbare medizinische Vorrichtung ausgewählt ist aus Herzunterstützungsvorrichtungen, Kathetern, Stents, prothetischen Implantaten, künstlichen Schließmuskeln und Arzneistoffabgabevorrichtungen, oder
wobei dieser biologisch aktive Wirkstoff einheitlich durch diese Matrixbeschichtung hindurch verteilt ist, oder
wobei dieser biologisch aktive Wirkstoff in dieser Matrixbeschichtung gelöst ist, oder
wobei dieser Schritt des Beschichtens Auftragen dieser Matrixbeschichtung in einer Dicke von 0,1 bis 5 Mikron umfasst, oder
wobei diese unbeschichtete implantierbare medizinische Vorrichtung beschichtet wird, um eine beschichtete implantierbare medizinische Vorrichtung herzustellen, wobei diese beschichtete implantierbare medizinische Vorrichtung bei Implantation in ein Tier reduzierte Proteinablagerung, reduzierte Fibrinogenablagerung, reduzierte Blutplättchenablagerung oder reduzierte inflammatorische Zelladhäsion im Vergleich zu dieser unbeschichteten implantierbaren medizinischen Vorrichtung hat.

9. Implantierbare medizinische Vorrichtung von einem beliebigen der Ansprüche 1-5, wobei diese implantierbare medizinische Vorrichtung ein Stent ist und wobei dieser biologisch aktive Wirkstoff ausgewählt ist aus antiproliferativen Wirkstoffen und Rapamycin-Makroliden.

10. Stent von Anspruch 9, wobei diese Matrixbeschichtung weiterhin einen biologisch aktiven Wirkstoff umfasst, ausgewählt aus antiproliferativen Wirkstoffen wie Methotrexat, Trimetrexat, Gemcitabin, Vinblastin, Vincristin, E-toposid, Teniposid, Topotecan, Irinotecan, Camptothecin, 9-Aminocamptothecin, Paclitaxel, Docetaxel, Daunorubicin, Doxorubicin, Dactinomycin, Idarubicin, Bleomycin, Tamoxifen; und Rapamycin-Makroliden, wie Rapamycin, CCI-779, Everolimus und ABT-578.

11. Stent von Anspruch 9 oder 10 zum Hemmen von Restenose an einer Stelle in einem Gefäß.

12. Implantierbare medizinische Vorrichtung von einem beliebigen der Ansprüche 1-5 zur Abgabe eines biologisch aktiven Wirkstoffs an ein Subjekt.

## Revendications

1. Dispositif médical implantable ayant une surface et un revêtement de matrice appliqué sur ladite surface dudit dispositif médical implantable, ledit revêtement de matrice se composant de constituants ayant un poids moléculaire inférieur à 20 kDa, ledit revêtement de matrice comprenant
(i) un oligomère ayant une structure décrite par la formule (II) :
F_{T}-[B-(oligo)]ₙ-B-(F_{T})_{g} (II)
dans laquelle
B comprend un uréthane ;
oligo comprend un oxyde de polypropylène, un oxyde de polyéthylène, un polycarbonate ou un oxyde de polytétraméthylène ;
F_{T} est un groupe oligofluoré ;
g est 1 ; et
n est un nombre entier de 1 à 10 ;
et
(ii) un agent biologiquement actif, dans lequel
(a) ledit revêtement de matrice est auto-éliminé ou biodégradable lors de l'implantation dans un sujet,
(b) ledit agent biologiquement actif, lorsque qu'il est sur le dispositif médical implantable est uniquement présent dans ledit revêtement de matrice ;
(c) ledit revêtement de matrice n'a pas les propriétés d'un polymère de base ;
(d) ledit revêtement de matrice est appliqué sur ladite surface par revêtement par atomisation, impression ou revêtement par immersion dudit dispositif médical implantable ; et
(e) ladite épaisseur du revêtement de matrice est de 0,05 à 15 µm.

2. Dispositif médical implantable selon la revendication 1, dans lequel ladite surface est un matériau choisi parmi les métaux, les alliages métalliques, les céramiques, les polymères de base, et les verres, ou
dans lequel ledit agent biologiquement actif est choisi parmi les protéines, les peptides, les glucides, les antibiotiques, les agents antiprolifératifs, les macrolides de type rapamycine, les analgésiques, les anesthésiques, les agents antiangiogéniques, les agents antithrombotiques, les agents vasoactifs, les anticoagulants, les immunomodulateurs, les agents cytotoxiques, les agents antiviraux, les anticorps, les neurotransmetteurs, les médicaments psychoactifs, les oligonucléotides, les vitamines, les lipides et des promédicaments de ceux-ci.

3. Dispositif médical implantable selon la revendication 1, dans lequel ledit agent biologiquement actif est complexé audit oligomère.

4. Dispositif médical implantable selon l'une quelconque des revendications 1 à 3, dans lequel F_{T} a la formule :
CF₃(CF₂)ₚX,
(CF₃)₂CF(CF₂)ₚX,
ou
(CF₃)₃C (CF₂)ₚX,
où X est CH₂CH₂-, (CH₂CH₂O)ₙ, CH₂CH(OH)CH₂O-, CH₂CH(CH₂OH)O-, ou une liaison ;
p est un nombre entier compris entre 2 et 20; et
n est un nombre entier compris entre 1 et 10.

5. Dispositif médical implantable selon l'une quelconque des revendications 1 à 4, dans lequel ledit dispositif est choisi parmi les dispositifs d'assistance cardiaque, les cathéters, les stents, les implants prothétiques, les sphincters artificiels, et les dispositifs de délivrance de médicaments, ou
dans lequel ledit agent biologiquement actif est distribué uniformément sur ledit revêtement de matrice, ou dans lequel ledit agent biologiquement actif est dissous dans ledit revêtement de matrice, ou dans lequel ledit revêtement de matrice a une épaisseur de 0,1 à 5 µm.

6. Procédé de préparation d'un dispositif de revêtement implantable revêtu selon l'une quelconque des revendications 1 à 5 ayant une surface, ledit procédé comprenant l'étape de revêtement de ladite surface avec un revêtement de matrice se composant de constituants ayant un poids moléculaire inférieur à 20 kDa, ledit revêtement de matrice comprenant (i) un oligomère et (ii) un agent biologiquement actif,
dans lequel ledit revêtement de matrice est auto-éliminé ou biodégradable lors de l'implantation dans un sujet et dans lequel ledit agent biologiquement actif, lorsque qu'il est sur le dispositif médical implantable est uniquement présent dans ledit revêtement de matrice.

7. Procédé selon la revendication 6, dans lequel ledit oligomère, lorsque qu'il est sur le dispositif médical implantable est uniquement présent dans ledit revêtement de matrice.

8. Procédé selon la revendication 6 ou 7, dans lequel ladite étape de revêtement comprend le brossage, l'impression, la pulvérisation, le revêtement avec un chiffon, ou l'immersion de ladite surface avec ledit revêtement de matrice, ou
dans lequel ladite étape de revêtement comprend la dissolution des constituants dudit revêtement de matrice dans un solvant pour former une solution et l'application de ladite solution sur la surface dudit dispositif médical implantable, ou
dans lequel ladite étape de revêtement comprend le mélange des constituants de ladite matrice avec un diluant pour former un mélange liquide et l'application dudit mélange liquide sur la surface dudit dispositif médical implantable, ou
dans lequel ledit dispositif médical implantable est choisi parmi les dispositifs d'assistance cardiaque, les cathéters, les stents, les implants prothétiques, les sphincters artificiels, et les dispositifs de délivrance de médicaments, ou
dans lequel ledit agent biologiquement actif est distribué uniformément sur ledit revêtement de matrice, ou
dans lequel ledit agent biologiquement actif est dissous dans ledit revêtement de matrice, ou
dans lequel ladite étape de revêtement comprend l'application dudit revêtement de matrice selon une épaisseur de 0,1 à 5 µm, ou
dans lequel ledit dispositif médical implantable non revêtu est revêtu pour produire un dispositif médical implantable revêtu, ledit dispositif médical implantable revêtu ayant, lors de son implantation dans un animal, un dépôt de protéines réduit, un dépôt de fibrinogènes réduit, un dépôt de plaquettes réduit, ou une adhérence de cellules inflammatoires réduite par comparaison avec ledit dispositif médical implantable non revêtu.

9. Dispositif médical implantable selon l'une quelconque des revendications 1 à 5, dans lequel ledit dispositif médical implantable est un stent, et dans lequel ledit agent biologiquement actif est choisi parmi les agents antiprolifératifs et les macrolides de type rapamycine.

10. Stent selon la revendication 9, dans lequel ledit revêtement de matrice comprend en outre un agent biologiquement actif choisi parmi les agents antiprolifératifs, tels que le méthotrexate, le trimétrexate, la gemcitabine, la vinblastine, la vincristine, l'étoposide, le téniposide, le topotécan, l'irinotécan, la camptothécine, la 9-aminocamptothécine, le paclitaxel, le docétaxel, la daunorubicine, la doxorubicine, la dactinomycine, l'idarubicine, la bléomycine, le tamoxifène ; et les macrolides de type rapamycine, tels que la rapamycine, CCI-779, l'évérolimus et ABT-578.

11. Stent selon la revendication 9 ou 10, pour l'inhibition de la resténose au niveau d'un site dans un vaisseau.

12. Dispositif médical implantable selon l'une quelconque des revendications 1 à 5, pour l'administration d'un agent biologiquement actif à un sujet.
